# EUROPEAN PATENT SPECIFICATION

(11) **EP 3 802 554 B1**
(45) Date of publication and mention of the grant of the patent: **13.12.2023**
(21) Application number: 19812121.2
(22) Date of filing: 21.05.2019
(51) Int. Cl.: C07F 9/53, C07F 9/6506, C07K 1/06, C07B 51/00

(54) **METHOD FOR SOLUTION-PHASE PEPTIDE SYNTHESIS AND PROTECTING STRATEGIES THEREFORE**
VERFAHREN FÜR PEPTIDSYNTHESE IN DER LÖSUNGSPHASE UND SCHUTZSTRATEGIEN DAFÜR
PROCÉDÉ DE SYNTHÈSE PEPTIDIQUE EN PHASE SOLUTION ET STRATÉGIES DE PROTECTION ASSOCIÉES

(30) Priority: 31.05.2018 US 201862678564 P
(43) Date of publication of application: 14.04.2021
(73) Proprietor: SEDERMA, 78610 Le-Perray-en-Yvelines (FR)
(72) Inventor: SEIFERT, Cole, 690 Rosebury Rd Helena, AL 35080 (US)
(74) Representative: de Saint Viance, Isabelle Marie Fanny
(86) International application number: PCT/US2019/033296
(87) International publication number: WO 2019/231760

(56) References cited:
- WO-A1-2017/112809
- US-A1- 2008 287 649
- US-A1- 2014 178 302
- US-A1- 2016 257 725
- Cole W. Seifert ET AL: "GAP Peptide Synthesis through the Design of a GAP Protecting Group: An Fmoc/ t Bu Synthesis of Thymopentin Free from Polymers, Chromatography and Recrystallization", European Journal of Organic Chemistry, vol. 2016, n.9, 1 March 2016 (2016-03-01), pages 1714-1719, XP055651402, DOI: 10.1002/ejoc.201600026 Retrieved from the Internet: URL:https://www.ncbi.nlm.nih.gov/pmc/artic les/PMC5486986/pdf/nihms837137.pdf [retrieved on 2019-12-10]
- Anonymous: "4-(Hydroxymethyl)phenoxyacetic acid | 68858-21-9", ChemicalBook >> CAS DataBase List, 1 January 2017 (2017-01-01), pages 1-4, XP055878431, Retrieved from the Internet: URL:https://www.chemicalbook.com/ChemicalP roductProperty_EN_CB0294351.htm [retrieved on 2022-01-13]
- GÓNGORA-BENÍTEZ MIRIAM ET AL: "Handles for Fmoc Solid-Phase Synthesis of Protected Peptides", ACS COMBINATORIAL SCIENCE, vol. 15, no. 5, 13 May 2013 (2013-05-13), pages 217-228, XP055878433, US ISSN: 2156-8952, DOI: 10.1021/co300153c Retrieved from the Internet: URL:http://www.ub.edu/chembiolab/pdf/ACSCo mbSci_2013,%2015,%20217.pdf>

## Description

### BACKGROUND OF THE INVENTION

Recent research efforts have made significant advancements in the area of purification chemistry, focusing specifically on avoiding column chromatography and recrystallization. This research has been defined as Group-Assisted Purification (GAP) chemistry/technology as a chemistry for organic synthesis that avoids traditional purification methods such as chromatography and/or recrystallization by purposefully introducing a well-functionalized group in the starting material or in the newly generated product. These GAP groups can also often be used as protecting groups to prevent undesired side-reactions during the synthesis of target molecules. Such research has the potential to encompass the entire field of synthetic organic chemistry.

Protecting groups are found in almost every complex synthesis where multiple functional groups are present. In regards to GAP chemistry, an ideal example would be one in which a semi-permanent protecting group introduced needed solubility characteristics required for GAP. However, most traditional protecting groups are nonpolar, and therefore do not generate the required GAP solubility for most substrates. If a protecting group could be developed that generated adequate solubility control, then GAP chemistry could potentially be extended to all syntheses which require the use of that protecting group. Several approaches have been utilized. Published patent application WO 2014093723 A2, teaches the protection of imines with a GAP-equipped chiral auxiliary, then using these chiral, N-phosphonyl imines as electrophiles in asymmetric boron addition reactions. Purification was conducted via GAP processes. This work is valuable in that it provides facile access to chiral, α-boronic acid amines, which could potentially be used to synthesize novel amino acid derivatives, which could potentially be incorporated into novel peptide targets.

Effective protecting groups need to be robust to a wide variety of conditions and must be added and removed with high yield. Protecting groups are used extensively in peptide synthesis, either for solid or solution phase approaches. For traditional peptide synthesis protection strategies, one of the most commonly used strategies is Fmoc/tBu. U.S. Patent No. 8,383,770 B2 teaches the use of the Fluorenylmethoxycarbonyl (Fmoc) and tert-Butyloxycarbonyl (Boc) N-terminus protecting groups in Solid-Phase Peptide Synthesis (SPPS). This technology is well known and widely applied in industry. The Fmoc group protects the N-terminus of amino acids to be added to the growing peptide, and tert-butyl (tBu) based groups protect the side chains of the same amino acids. The Fmoc group can be removed with a proper deprotection base while the tBu groups remain until acid-based global deprotection at the end of the synthesis. Boc and Fmoc groups have been used for decades in all areas of peptide chemistry, and the preferred Fmoc group is almost entirely restricted to SPPS.

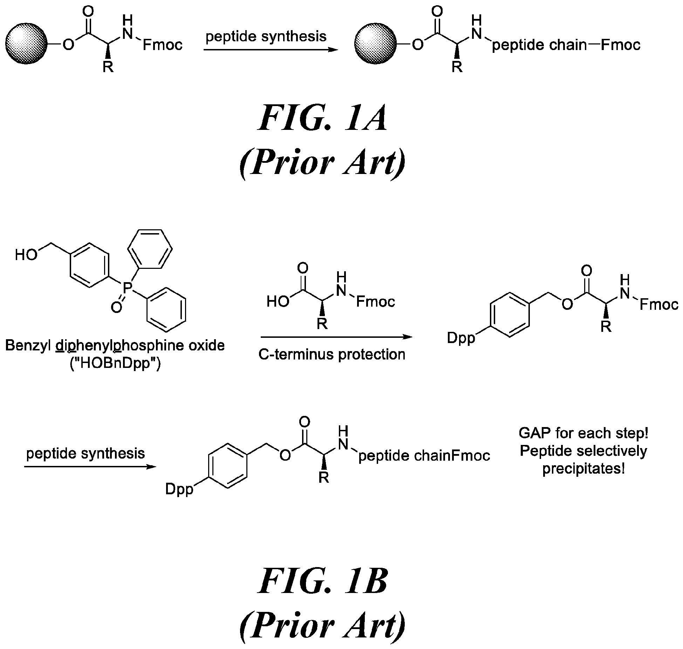

Developed by Merrifield in the 1960s, SPPS has become a standard protocol used by multiple scientific disciplines for research and manufacturing (see **FIG 1A**). The advantages of the polymer support or resin lie in its ability to allow facile purification of the growing peptide after each coupling/deprotection step, which avoids the use of column chromatography. The key disadvantage of SPPS lies in the difficulty of scale-up: many polymer supports are expensive and occupy the vast majority of the mass of the material to be worked with. Coupling reactions in SPPS are also inefficient because the reactions occur on a solid-liquid interface. Additionally, after each deprotection and coupling reaction, the resin must be washed with solvent to remove any impurities generated from previous reactions, and this generates significant solvent waste that can be extremely problematic on a large scale.

Examples of economically feasible Fmoc protection schemes for solution-phase peptide synthesis (SolPPS), however, are rare, with few examples in the literature at all. U.S. Patent No. 5,516,891 A provides one of the few examples of Fmoc-based SolPPS. Again, the Fmoc peptide synthesis is almost entirely restricted to SPPS, due to the formation of N-fluorenylmethylpiperidine (NFMP) as a side product during deprotection, which is difficult to remove without polymer supports. The standard protocol for Fmoc deprotection is to stir the Fmoc-peptide in a solution of dimethylformamide (DMF) or dichloromethane (DCM) with excess piperidine, deprotecting the Fmoc group and forming NFMP in the process. The '891 patent teaches removal of this impurity by deprotecting with 4-aminomethylpiperidine (4AMP) instead of piperidine. This forms NFMP-CH₂NH₂ instead of NFMP, which due to the presence of the extra amino group, can be extracted into water. The problem with this method lies in the high cost of using 4AMP. This is why this method is cost prohibitive, and why it has not been accepted by the industry.

Another example of Fmoc-based SolPPS can be seen in published patent application WO2017112809A1. This publication teaches the use of a C-terminus GAP protecting group, benzyl diphenylphosphine oxide (HOBnDpp), to control the solubility of the target peptide to allow for selective precipitation after each successive coupling reaction (see **FIG 1B**). The solubility is controlled such that the growing peptide remains in an organic solvent, such as ethyl acetate or DCM, and aqueous washes are performed to remove impurities; a subsequent concentration of the organic solvent followed by mixture in an alkane solvent selectively precipitates the peptide product. While this technology adapted Fmoc/tBu chemistry to solution-phase in a much more economically feasible manner than the '891 patent, there are potential limitations inherent in the method. First, the GAP group is not acid labile, preventing convenient one-step global deprotection with commonly used trifluoroacetic acid-based (TFA-based) or other acidic cocktails; hydrogenation or hydrolysis is required to remove HOBnDpp from the C-terminus of the peptide. Additionally, as the peptide continues to grow and change in sequence, HOBnDpp maintains less and less solubility control over the chain as it becomes longer, or as sequences lend different solubility characteristics to the peptide. HOBnDpp is also prone to hydrolysis or accidental cleavage during the synthesis. For example, during Fmoc deprotection of the second amino acid in the peptide sequence, diketopiperazine (DKP) formation facilitates the loss of HOBnDpp from the C-terminus. This cleaved HOBnDpp remains in the reaction solution and can react with activated amino acids during coupling reactions to generate impurities that are difficult or impossible to remove. Moreover, with the current cleavage techniques (either hydrolysis or hydrogenation) for deprotection of the C-terminus after the protected peptide is synthesized, the C-terminus of the final peptide product is a carboxylic acid. Many therapeutic peptides conversely require the C-terminus to be modified, such as into an amide, a cyclic peptide, a thioester, etc., in lieu of this carboxylic acid, further limiting the application of the GAP peptide synthesis method. Additionally, these cleavage reactions to remove HOBnDpp from the C-terminus are completely separate reactions from the global deprotection; the reactions require specific conditions and work ups, an inconvenience that costs both time and money.

The scientific publication "GAP Peptide Synthesis through the Design of a GAP Protecting Group: An Fmoc/ t Bu Synthesis of Thymopentin Free from Polymers, Chromatography and Recrystallization", European Journal of Organic Chemistry, val. 2016, n.9, 2016-03-01, pages 1714-1719, Cole W. Seifert et Al. discloses the method for SolPPS using Fmoc/Boc protecting groups with the benzyl-type GAP protecting group HOBnDpp utilized in place of a polymer support, facilitating C to N Fmoc peptide synthesis without chromatography, recrystallization, or polymer supports.

HMPA (4-(Hydroxymethyl)phenoxyacetic acid, CAS No 68858-21-9) is disclosed as a linkage agent used in solid-phase peptide synthesis according to the "FMOC-polyamide" technique.

The scientific publication "Handles for Fmoc Solid-Phase Synthesis of Protected Peptides", ACS Combinatorial Science, vol. 15, no. 5, 13 May 2013 (2013-05-13), pages 217-228, Góngora-Benítez Miriam et al., is an overview of acid-labile linkers for protected peptide synthesis on solid phase.Because of these and other issues with scale up in SPPS, as well as the limitations in the existing GAP peptide synthesis protection strategy, there is a need in the industry for not just SolPPS methods amenable to scale up, but SolPPS methods that are more amenable to (i) various cleavage conditions, (ii) longer or more difficult (from a solubility perspective) sequences, (iii) and C-terminus modification.

### SUMMARY OF THE INVENTION

In one aspect, the invention disclosed herein comprises a protecting group for solution-phase peptide synthesis, the protecting group having a chemical formula selected from the group consisting of Chemical Formula 1, Chemical Formula 2, Chemical Formula 3, Chemical Formula 4, Chemical Formula 5, Chemical Formula 6, Chemical Formula 7, and Chemical Formula 8.
Chemical Formula 1 is: 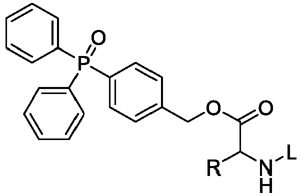
Chemical Formula 2 is: 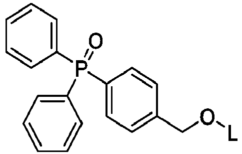
Chemical Formula 3 is: 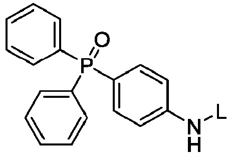
Chemical Formula 4 is: 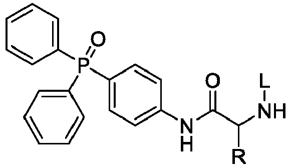
Chemical Formula 5 is: 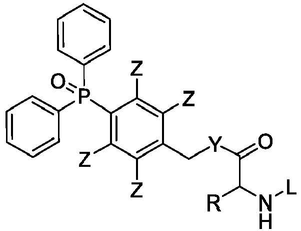
Chemical Formula 6 is: 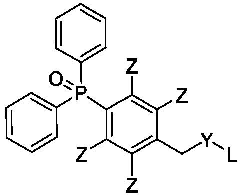
Chemical Formula 7 is: 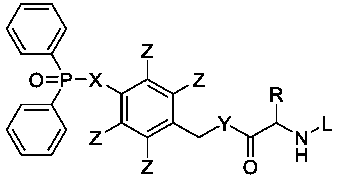 ; and
Chemical Formula 8 is: 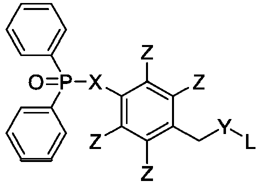
Z is selected from the group consisting of: -H, a methyl group (-Me), and a methoxy group (-OMe). Y is selected from the group consisting of: -O-, -S-, and -NH-. X is selected from the group consisting of: -O-, -S-, and -NH-. R is selected from the group consisting of known side chains of protected amino acids and known side chains of unprotected amino acids. L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

In a second aspect, the disclosed invention comprises a method of performing solution-phase peptide synthesis, wherein the method comprises several steps. An initial step comprises attaching a first protecting group to a first amino acid to provide a first protected amino acid. A subsequent step comprises performing a coupling reaction on the first protected amino acid or a peptide formed by linking the first amino acid to another molecule. The first protecting group is the protecting group of claim 1.

In a third aspect, the disclosed invention comprises a method of forming a protecting group for solution-phase peptide synthesis. The method comprises coupling benzyl diphenylphosphine oxide (HOBnDpp), aniline diphenylphosphine oxide (NH₂PhDpp), or a derivative thereof (i) directly to a linker molecule, (ii) to a linker molecule via an amino acid, or (iii) to a linker molecule via an amino acid derivative.

### BRIEF DESCRIPTION OF THE DRAWINGS

The foregoing and other objects, features, and advantages of the disclosure will be apparent from the following description of embodiments as illustrated in the accompanying drawings, in which reference characters refer to the same parts throughout the various views. The drawings are not necessarily to scale, emphasis instead being placed upon illustrating principles of the disclosure. The figures are used as non-limiting examples, only intended to portray preferred embodiments without limiting the scope of this disclosure:
FIG 1A depicts a prior art process of Solid Phase Peptide Synthesis (SPPS).
FIG 1B depicts steps of the GAP Peptide Synthesis (GAP-PS) process, specifically the use of a HOBnDpp for C-terminus protection.
FIG 2 depicts a process for attaching the protecting group of FIG 1B to the side chain of an amino acid residue, specifically serine in this non-limiting embodiment, followed by GAP-PS.
FIG 3 depicts a schematic for synthesizing, as an example, a novel GAP molecule and attaching it to a peptide, wherein HOBnDpp is reacted with protected Rink Amide-OH and the resulting new chemical entity Rink Amide-GAP (RAG) is attached to the C-terminus of an amino acid.
FIG 4 depicts the attachment of a novel GAP molecule to the side chain of an amino acid. In this non-limiting example, the GAP molecule RAG is used to protect the side chain of aspartic acid, resulting in synthesis of RAG-protected asparagine. Specifically, a coupling reaction is performed to form an amide bond between the free amine on the RAG molecule and the carbonyl carbon of asparagine.
FIG 5 depicts a non-limiting example of the synthesis of a novel GAP molecule. In this non-limiting example, HOBnDpp is connected to protected HMPA-OH directly to form HMPA-GAP (HG), as well as through an intermediary (phenylalanine in one embodiment) to form the GAP molecule, HMPA-phenylalanine-GAP (HPG). Specifically, HOBnDpp can be coupled to the carboxylic acid moiety of protected HMPA-OH, or HOBnDpp can be coupled initially to an amino acid followed by coupling to HMPA to create the GAP molecule HPG.
FIG 6 depicts the attachment of HPG from FIG 5 to the C-terminus of a generic amino acid, as well as to, in a preferred embodiment of this disclosure, the side chain of aspartic acid. Specifically, this reaction is a coupling reaction that can be accomplished through the use of multiple different coupling reagents, wherein the free alcohol of HPG couples to the carbonyl group of an activated carboxylic acid.
FIG 7 depicts a non-limiting example of the synthesis of a novel GAP molecule, aniline diphenylphosphine oxide ("NH₂PhDpp").
FIG 8 depicts a non-limiting example of the synthesis of a novel GAP molecule, wherein NH₂PhDpp is reacted with protected Rink Amide-OH to achieve the target entity. Specifically, NH₂PhDpp can be coupled to the carboxylic acid moiety of protected Rink Amide-OH via a multitude of coupling reagents.
FIG 9 depicts non-limiting examples of the synthesis of a novel GAP molecule, wherein NH₂PhDpp is reacted with protected HMPA-OH, either directly or through an intermediary, to form the target molecule. Multiple different coupling reagents can be used to facilitate these reactions.
FIG 10 shows, as a non-limiting example, the resulting compound of the attachment of the HPG GAP molecule to the C-terminus of Fmoc-protected leucine.
FIG 11 depicts as a non-limiting example the compound resulting from the side-chain protection of glutamic acid with HPG, wherein the C-terminus of glutamic acid is protected with an allyl group as a non-limiting example of possible C-terminal protection strategies.
FIG 12 shows, as a non-limiting example, the tetra-peptide, Fmoc-Glu(HPG)-Glu(HPG)-Tyr(tBu)-Leu-HPG, resulting from the novel SolPPS method disclosed herein.
FIG 13 depicts the compound resulting from the attachment of RAG to the side chain of an amino acid, specifically aspartic acid in this non-limiting example, wherein the C-terminus of aspartic acid is protected with an allyl group as a non-limiting example of C-terminal protection strategies.
FIG 14 shows as a non-limiting example the protected peptide resulting from the SolPPS method disclosed herein.
FIG 15 depicts, as a non-limiting example, the peptide bivalirudin fully protected with novel GAP molecules.
FIG 16 depicts fully deprotected bivalirudin, attained through a one-step global deprotection that removes all side-chain and C-terminal protecting groups subsequent to Fmoc-deprotection.
FIG 17 shows a general methodology for protecting amines with novel GAP molecules. In this particular non-limiting embodiment, HPG is used.
FIG 18 shows as non-limiting examples a myriad of molecules that can serve as reagents for the synthesis of novel GAP molecules disclosed herein.

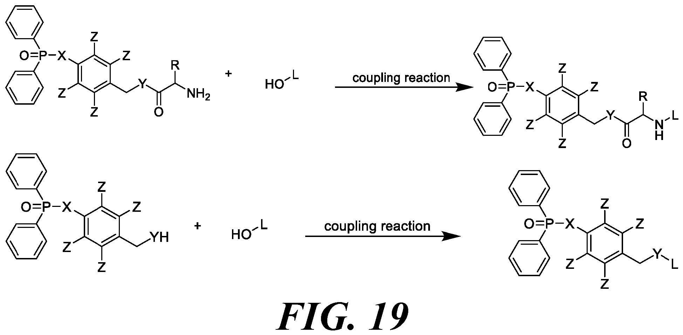

FIG 19 shows a myriad of representative coupling reactions to synthesize novel GAP molecules disclosed herein.

### DETAILED DESCRIPTION OF THE DISCLOSURE

In the Summary of the Invention above and in the Detailed Description of the Invention, and the claims below, and in the accompanying drawings, reference is made to particular features of the invention. It is to be understood that the disclosure of the invention in this specification includes all possible combinations of such particular features. For example, where a particular feature is disclosed in the context of a particular aspect or embodiment of the invention, or a particular claim, that feature can also be used, to the extent possible, in combination with and/or in the context of other particular aspects and embodiments of the invention, and in the invention generally.

The term "comprises" and grammatical equivalents thereof are used herein to mean that other components, ingredients, steps, etc. are optionally present. For example, an article "comprising" (or "which comprises") components A, B, and C can consist of (i.e., contain only) components A, B, and C, or can contain not only components A, B, and C but also one or more other components.

Where reference if made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

The term "at least" followed by a number is used herein to denote the start of a range beginning with that number (which may be a range having an upper limit or no upper limit, depending on the variable being defined). For example, "at least 1" means 1 or more than 1. The term "at most" followed by a number is used herein to denote the end of a range ending with that number (which may be a range having 1 or 0 as its lower limit, or a range having no lower limit, depending upon the variable being defined). For example, "at most 4" means 4 or less than 4, and "at most 40%" means 40% or less than 40%. When, in this specification, a range is given as "(an initial number) to (a subsequent number)" or "(an initial number)-(a subsequent number)," this means a range whose lower limit is the initial number and whose upper limit is the subsequent number. For example, 25 to 100 mm means a range whose lower limit is 25 mm, and whose upper limit is 100 mm.

The term "first" is used to distinguish one element from another element and is not meant denote that an element is the primary or initial element in any given sequence of elements. For example, "a first amino acid" does not signify that the amino acid is the first in a sequence of amino acids or the first amino acid to be reacted. Instead, "a first amino acid" only indicates that the amino acid is separate and distinguishable from another amino acid, such as "a second amino acid."

The term "coupling reaction" is used to refer generally to the formation of a bond between two constituent molecules facilitated by a "coupling reagent." In peptide chemistry, these coupling reactions can occur via many different mechanisms under many different reaction conditions that can completely depend on the coupling reagent used. For example, a coupling reagent can "activate" the carboxylic acid of a constituent molecule such that the carbonyl carbon can be more prone to nucleophilic attack. Coupling reactions can result in the loss of a water molecule during the formation of the bond between the two constituent molecules (see Chandrudu 2013, Mollica 2013, Shelton 2013, Amblard 2006, Bachem 2016).

In many types of protecting schemes for peptide synthesis, a repetition of similar reactions occurs to grow the peptide chain. Generally, either the N- or C-terminus of each amino acid added to the chain is initially protected, and the other terminus of the amino acid is free to participate in a coupling reaction. After addition to the chain via the initially-free terminus, a deprotection reaction is run, freeing up the protected N- or C-terminus to participate in a subsequent coupling reaction to create a peptide bond with the next amino acid. For example, in Fmoc/tBu-based peptide synthesis, the Fmoc group protects the N-terminus of amino acids, and side chains of amino acids are protected with tBu-based protecting groups, including but not limited to butyl, trityl (triphenylmethyl), Boc (butyloxycarbonyl), Pbf (2,2,4,6,7-pentamethyl-2,3-dihydrobenzofuran-5-sulfonyl), Pmc (2,2,5,7,8-pentamethylchromane-6-sulfonyl), and Acm (acetamidomethyl) (some amino acids do not require side-chain protection because the side-chains are naturally inert to coupling and deprotection conditions). The C-terminus of the primary amino acid in the peptide sequence is connected to and protected by a resin or polymer in SPPS, and a protecting group in SolPPS. The Fmoc/tBu peptide synthesis scheme is designed such that the Fmoc group on the N-termini of amino acids is base-labile, and treatment with the proper deprotection base removes the Fmoc group from the N-termini without interfering with any C-terminus connections or side-chain protections. Once the deprotection reaction is performed, the N-terminus of the primary amino acid is free, while the C-terminus and side chain are protected or otherwise inert. Then, the next amino acid, with the N-terminus Fmoc-protected and the side chain protected or naturally inert, is activated at the free C-terminus with a coupling reagent, and such activation facilitates nucleophilic attack by the free N-terminus of the primary amino acid on the activated carbonyl to form a peptide bond between the primary and next amino acid. This process is repeated until the proper peptide sequence is achieved. After Fmoc deprotection of the final amino acid, the peptide is still protected at the C-terminus and at the side chains. A global deprotection with a strong acid cocktail such as a TFA-based cocktail is then performed to remove all of the side-chain protecting groups; in some cases, the C-terminal resin or protecting group can also be cleaved.

The present disclosure addresses failings in the art by providing a method for solution-phase peptide synthesis that allows for various global deprotection strategies, the synthesis of longer or more difficult (solubility-wise) peptide sequences, and C-terminus modifications on target peptides while maintaining solubility control of the target peptide and overall synthesis process . By utilizing novel protection strategies on the C-terminus and side chains of the growing peptide, a SolPPS strategy is presented that is economically feasible and useful for the commercial production of peptides.

It is therefore a non-limiting object of the present disclosure to enable a novel method of SolPPS. In one aspect, HOBnDpp, traditionally a C-terminus GAP protecting group, is used to directly protect the side chains of amino acid residues, including, but not limited to, serine, threonine, tyrosine, or cysteine, in lieu of or in addition to the C-terminus of the peptide chain. The other amino acid residues of the desired peptide can be protected using a number of different protection strategies, including, but not limited to, Fmoc/tBu, Boc/benzyl, Cbz (benzyloxycarbonyl)/tBu, Cbz/benzyl, etc. This enhanced protection strategy allows for the synthesis of longer and/or more difficult sequences while maintaining solubility control of the peptide chain. This strategy also enables the use of different C-terminus protecting groups for solubility, deprotection, or post-synthesis C-terminus modification purposes. In another aspect, novel GAP molecules as discussed below are used to protect the C-terminus and/or side chains of certain amino acids to allow for peptide synthesis in solution in the C to N direction.

In another aspect, a novel SolPPS method is accomplished through specially designed C-terminal protecting strategies, wherein novel GAP molecule protecting groups maintain benefits of original GAP peptide synthesis (GAP-PS) while allowing for a wider range of cleavage conditions, the synthesis of longer peptides, minimized unintentional or accidental deprotection, and C-terminal modifications upon cleavage. Depending on the GAP molecule used, C-terminus deprotection can be accomplished under either acidic conditions, including but not limited to treatment with trifluoroacetic acid (TFA), hydrofluoric acid (HF), toluenesulfonic acid (TsOH), methanesulfonic aicd (MsOH) or others, as well as basic hydrolysis conditions, including but not limited to metal hydroxides in either aqueous or alcoholic systems, or bi-phasic systems with phase-transfer catalysts, as well as reductive conditions, including but not limited to molecular hydrogen over transition metal catalysts such at Pd⁰ or Pt⁰ or Ru, transfer hydrogenation, or other hydrogen sources such as borohydrides, aluminum hydrides, silanes, ammonium formate, etc. Also depending on the specific GAP molecule, C-terminus amide formation or other post-synthesis modifications can be accomplished during the cleavage of the C-terminus GAP molecule, generating desired peptide products while avoiding extra post-synthesis processing steps. These novel GAP molecules can remain stable to acid-catalyzed deprotection reactions, as well as hydrogenation-based and hydrolysis reactions, allowing for increased post-peptide-synthesis recovery of the GAP protecting groups. Additionally, loss of C-terminal protection mid-synthesis is also minimized by these novel GAP molecules.

In another aspect, any of the novel GAP molecules described above are used to protect the side chains of various amino acids, including, but not limited to, aspartic acid, cysteine, serine, threonine, lysine, glutamic acid, asparagine, and glutamine, in lieu of or in addition to the C-terminus of the peptide. This strategy allows for the protection of reactive side chains while simultaneously controlling the solubility of the target peptide.

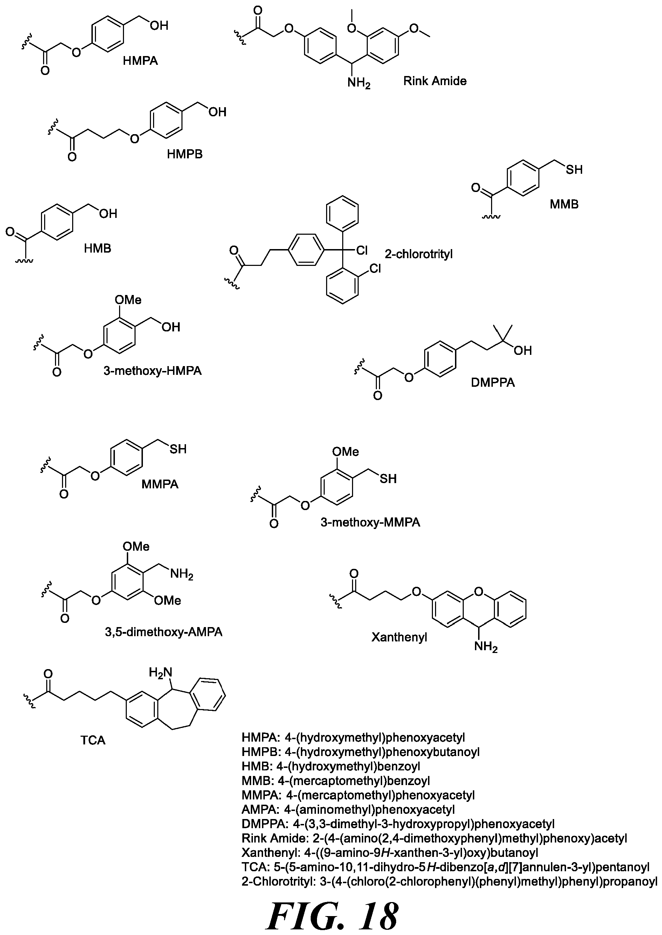

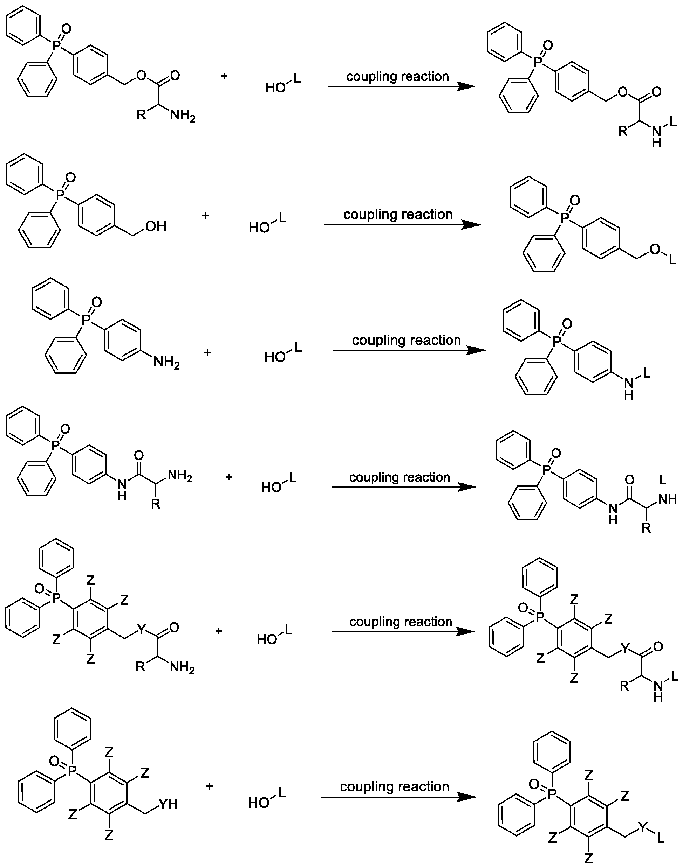

In another aspect, methods of synthesizing these novel GAP molecules and derivatives thereof are presented. As a non-limiting example, HOBnDpp is attached to carboxylic acid moiety of a linker molecule, examples of which can be seen in **FIG 18****,** either directly or through an intermediary, and a resulting novel GAP protecting group is then attached to the C-terminus of the peptide. As another non-limiting example, aniline diphenylphosphine oxide (i.e. NH₂PhDpp), is coupled to different linkers in a similar manner to achieve novel GAP protecting groups that can be used in a similar manner.

In another aspect, any of these different strategies discussed above are utilized in different combinations to enable the best possible synthesis strategy for a given peptide. As a non-limiting example, when used appropriately, these strategies allow for the synthesis of longer peptides entirely in solution, with C-terminus deprotection and C-terminus amide formation being accomplished along with side chain deprotection in a one-step reaction.

It is therefore a non-limiting object of the present disclosure to provide a method for SolPPS. In designing this method, it was apparent that the method should seek to maintain the advantages of other solution-phase methods like GAP-PS, such as avoiding arduous distillation and purification strategies that hinder large scale productions. An enhanced protection strategy would need to be designed to maintain solubility control of the target peptide to allow separation of the product from generated amino acid and deprotection protocol impurities, while also addressing the shortcomings in the art.

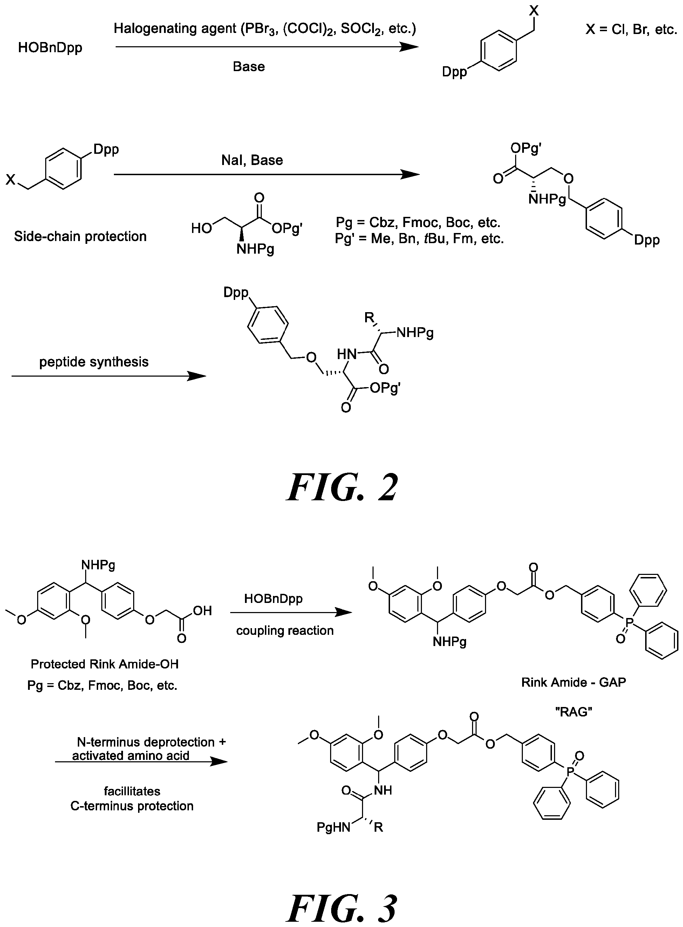

In a non-limiting exemplary embodiment of the present disclosure, a novel SolPPS method can begin with attachment of HOBnDpp directly to the C-terminus of the primary amino acid in a given sequence (see **FIG 1B**)**.** Alternatively, or in addition, to protecting the C-terminus of a primary amino acid with HOBnDpp, attachment of HOBnDpp to side chains of amino acid residues, such as, but not limited to, serine, threonine, tyrosine, and/or cysteine, with subsequent incorporation into the growing peptide, can also be executed to better control solubility and protect the side chains of certain amino acids (see **FIG 2**). To accomplish side-chain protection with HOBnDpp, HOBnDpp is initially reacted with a halogenating agent (for example, PBr₃, POBr₃, (COCl)₂, SOCl₂, etc.) in the presence of a base (for example, triethylamine, diisopropylethylamine, sodium bicarbonate, etc) to provide either IBnDpp, ClBnDpp or BrBnDpp, depending on the halogenating reagent used. The reaction can occur at a range of conditions depending on the halogenating agent and base used for the reaction. As an example, the reaction can be conducted at a pressure from 1 to 100 bar, at a temperature from -78 °C to 100 °C, and in mono-phasic or bi-phasic reaction medium. This halogenated compound can then be reacted with an amino acid with a primary protecting group (Pg) on the N-terminus and a secondary protecting group (Pg') on the C-terminus to effectively protect the side chain of the amino acid. Peptide synthesis can then be run in the C to N direction if a GAP molecule protects the C-terminus of the peptide in lieu of or in addition to GAP protection of the side chain of one or multiple amino acids to be integrated into the peptide sequence; alternatively, peptide synthesis can be run or in the N to C direction if HOBnDpp or other GAP molecule only protects an amino acid side chain. A myriad of protecting strategies for N-terminus and side-chain protection can be possible. Potential N-terminus or C-terminus protecting groups can include, but are not limited to, Cbz, Fmoc, Boc, methyl, Bn (benzyl), or any of the GAP molecules discussed herein, and side chain protecting groups can include, but are not limited to, tBu, Acm, Trityl, Boc, Pbf, Pmc, Fm (fluorenylmethyl), and any of the GAP molecules discussed herein.

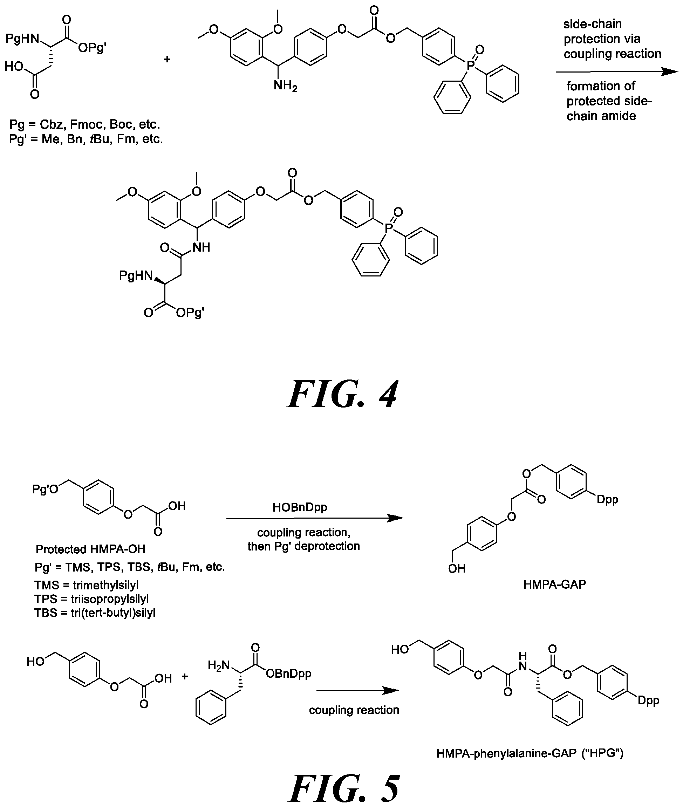

In another non-limiting exemplary embodiment, a novel SolPPS method can begin with a novel GAP molecule (here, RAG) being used for C-terminus protection as seen in **FIG 3****,** enabling both acid-labile C-terminus protection of the peptide and C-terminus amide formation upon cleavage of RAG from the peptide. Once C-terminal protection with RAG is accomplished, peptide synthesis can run in the C to N direction, utilizing a myriad of N-terminus and side-chain protection strategies, such as, but not limited to, the Fmoc/tBu strategy. To synthesize RAG, HOBnDpp can be coupled to protected Rink Amide-OH via a multitude of coupling reagents, such as, but not limited to, TFFH ("N-((dimethylamino)fluoromethylene)-N-methylmethanaminium hexafluorophosphate"), TBTU ("1-((dimethylamino)(dimethyliminio)methyl)-1H-benzo[d][1,2,3]triazole 3-oxide tetrafluoroborate"), HBTU ("1-((dimethylamino)(dimethyliminio)methyl)-1H-benzo[d][1,2,3]triazole 3-oxide hexafluorophosphate"), EDCI ("3-(((ethylimino)methylene)amino)-N,N-dimethylpropan-1-amine"), or COMU ("(1-Cyano-2-ethoxy-2-oxoethylidenaminooxy)dimethylamino-morpholino-carbenium hexafluorophosphate") to provide the GAP molecule RAG. The coupling reaction can occur at a range of conditions depending on the specific coupling reagent used. As an example, the reaction can be conducted at atmospheric pressure at room temperature with light stirring with TBTU. Once the protected RAG molecule is synthesized, a deprotection reaction can occur (for example, an Fmoc deprotection reaction) to free up the amine on the Rink Amide to participate in a coupling reaction to a carboxylic acid, such as can be found at the C-terminus of an amino acid. RAG can alternatively or additionally be attached to the side chains of amino acid residues, including, but not limited to, aspartic acid and glutamic acid (see **FIG 4**) to allow for enhanced protection or solubility control upon the addition of those amino acids to the growing sequence. Upon cleavage of RAG from the side chains of amino acids, amide-forms of those side chains (here, asparagine and glutamine, respectively) can be generated (see **FIG 4**)**.**

In another non-limiting exemplary embodiment, a novel SolPPS method can begin with a novel GAP molecule (here, (Rink Amide)-phenylalanine-GAP, i.e. RPG) being used for C-terminus protection. RPG can be synthesized similarly to **FIG** 5. Specifically, HOBnDpp may be coupled to Fmoc-protected phenylalanine to form Fmoc-phenylalanine-GAP (i.e. Fmoc-F-GAP), and after Fmoc deprotection, the now-free N-terminus of GAP-protected phenylalanine may be coupled the carboxylic acid moiety of protected Rink Amide-OH (the molecular structure of protected Rink Amide-OH may be seen in **FIG 3**). The resulting structure can then be deprotected at the N-terminus of the Rink Amide molecule, and the free N-terminus can then participate in a coupling reaction to form a peptide bond with the primary amino acid in a given peptide sequence. Peptide synthesis can then proceed in the C to N direction with RPG providing C-terminus protection of the growing peptide. RPG as a C-terminal protecting group can be used in conjunction with any of the disclosed GAP molecules that provide side-chain protection, or with any other protecting strategies available for N-terminus and side-chain protection. RPG can also be used to protect the side chains of amino acids just as RAG is used, either in addition to or in lieu of C-terminal protection.

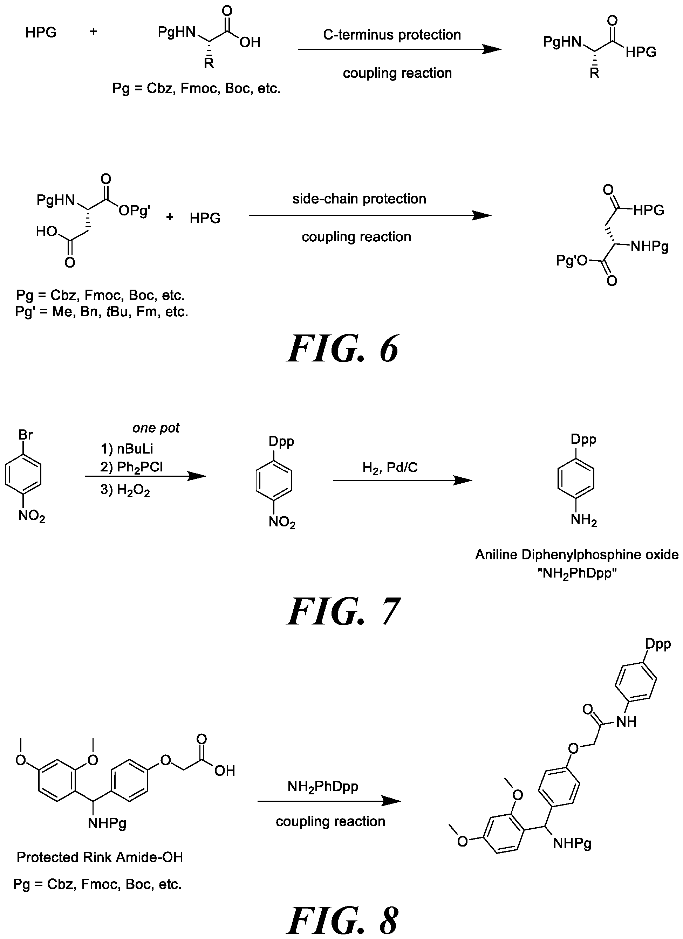

In another non-limiting example, a novel SolPPS method can utilize a different novel GAP molecule, such as HG or HPG, for C-terminus protection. Synthesis of these GAP molecules can be seen in **FIG 5****,** and these molecules can be attached to the C-terminus of a peptide to allow for acid-labile C-terminus protection without C-terminus amide formation upon cleavage (see **FIG 6**). In lieu of or in addition to protecting the C-terminus of amino acids, HG and/or HPG can also be attached to the side chains of amino acid residues, including, but not limited to, aspartic acid, cysteine, serine, threonine, tyrosine, and glutamic acid, to assist in solubility control (see **FIG 6**). Peptide synthesis can then proceed in the C to N direction utilizing a myriad of protection strategies, such as, but not limited to, the Fmoc/tBu strategy.

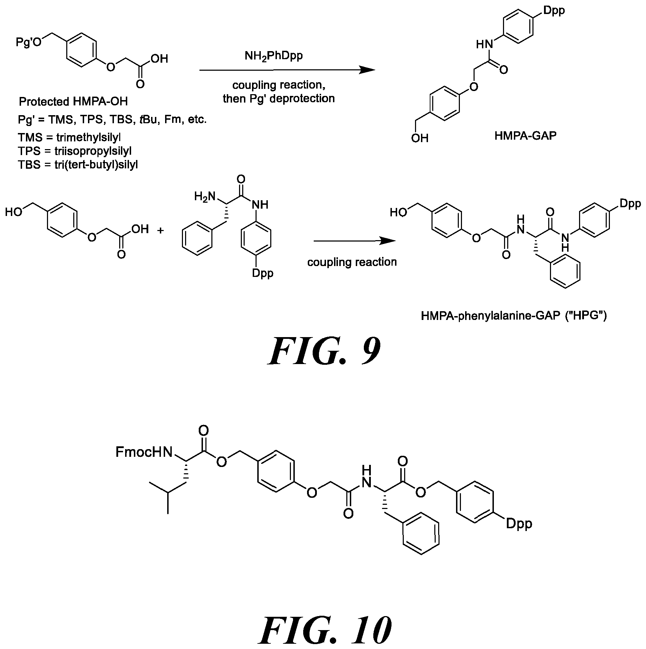

In another non-limiting exemplary embodiment, a novel SolPPS method can begin with the synthesis of aniline diphenylphosphine oxide (i.e. NH₂PhDpp) as seen in **FIG 7**. Specifically, synthesis of NH₂PhDpp begins with 4-bromonitrobenzene, which can be activated N-butyllithium (nBuLi) in ethereal solvent at ultra-low temperature (-100 to -30° C) and room pressure to form an organolithium reagent. This reagent can then react in-situ with a slow addition of chlorodiphenyl phosphine which, after phosphine oxidation with hydrogen peroxide and nitro group reduction with H₂ and 10% Pd/C, will afford NH₂PhDpp. This molecule can then be attached, in a non-limiting example, to Rink Amide-OH (see **FIGS 8****,** **18**), and the resulting GAP molecule can be attached to the C-terminus of a peptide similarly to **FIG 3** and/or the side chains of aspartic acid or glutamic acid (creating protected asparagine or glutamine, respectively) similarly to **FIG 4****.** In another non-limiting example, another novel GAP molecule can be synthesized via reaction of NH₂PhDpp with the carboxylic acid moiety of a linker (here, HMPA) either directly or through an amine-based intermediary as seen in **FIG 9****.** The resulting GAP molecule can then be attached to the C-terminus of the peptide and/or to the side chains of aspartic acid, cysteine, serine, threonine, and glutamic acid as seen in **FIG 6****.** Peptide synthesis can then proceed in the C to N direction utilizing a myriad of protection strategies, such as, but not limited to, the Fmoc/tBu strategy.

For all of the above non-limiting examples, HOBnDpp, NH₂PhDpp, or any other GAP molecule can be attached to the C-termini or to the side chains of amino acid residues in a number of different solvents, including, but not limited to, dichloromethane, ethyl acetate, isopropyl acetate, methyltetrahydrofuran, tetrahyrdrofuran, and propylene carbonate. For any of the coupling reactions described herein, whether for GAP molecule synthesis or attachment of a GAP molecule to an initial amino acid of a peptide to be synthesized, a myriad of different coupling reagents can be used, including, but not limited to, TFFH, TBTU, HBTU, CONW, or EDCI.

As a non-limiting exemplary embodiment, and with any of the above discussed examples or any combinations thereof, SolPPS can then be accomplished in the C to N direction, such as with Fmoc/tBu chemistry. Deprotection of the Fmoc group for the primary and subsequent amino acids can be carried out with a myriad of deprotection reagents, including diethylamine, DBU (diazabicycloundecene), piperidine, tertybutylamine, and other alkyl amines, and additional coupling reactions can be performed to grow the peptide chain. After each coupling reaction, different quenching agents can be used to nullify excess activated amino acids and lend desired solubility characteristics to the resulting molecule. In a non-limiting example, decylamine can be used to quench activated amino acid and increase the resulting molecule's solubility in alkane solvents. Subsequently, depending on the reaction solvent, liquid-liquid extraction techniques or selective precipitation can be performed to purify the reaction mixture. The novel GAP molecules disclosed herein allow for solubility control and enable a novel SolPPS method, wherein selective precipitation or extraction effectively removes amino acid and fulvene impurities (i.e., NFMP). After the last amino acid of the desired peptide sequence is coupled, in a non-limiting example, global deprotection with an appropriate TFA cocktail can be performed to remove the acid-labile GAP molecules from the C-terminus and appropriate side chains; such deprotection can also remove other acid-labile side-chain protecting groups, such as tBu-based side-chain protecting groups. This novel SolPPS method enables a one-step global deprotection to yield the native peptide sequence, avoiding additional reactions like hydrogenation or hydrolysis to remove the C-terminal protecting group. Additionally, if a GAP molecule such as RAG is used, the C-terminal amide can be formed during the TFA cleavage, successfully avoiding post-synthesis reactions that would normally be required for such modification. These novel GAP molecules disclosed herein additionally remain stable during peptide synthesis, with no accidental hydrolysis or removal due to DKP formation.

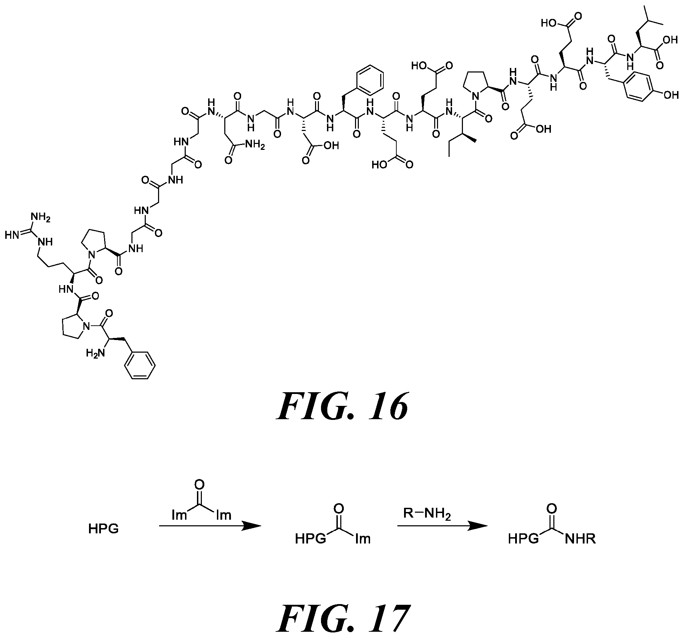

In one non-limiting embodiment, these novel GAP molecules can be used to protect the N-terminus of any given peptide, as well as the amine-based side chains of certain amino acid residues, including, but not limited to, lysine. In a non-limiting example, HPG and diimidazole carbonyl are dissolved in an appropriate reaction solvent, such as DCM, and lightly stirred at room temperature and pressure for one hour or more. An amine is then added to the reaction mixture at room temperature and pressure to yield an HPG-protected carbamate product (see **FIG 17**).

It another non-limiting embodiment, a novel SolPPS method can begin with the synthesis of novel GAP molecule protecting groups, wherein HOBnDpp, NH₂PhDpp, or a derivative thereof can be directly reacted with the carboxylic acid moiety of any given linker molecule, examples of which can be seen in **FIG 18****.** and in the table below:

| |
|---|
| HMPA: 4-(hydroxymethyl)phenoxyacetyl |
| HMPB: 4-(hydroxymethyl)phenoxybutanoyl |
| HMB: 4-(hydroxymethyl)benzoyl |
| MMB: 4-(mercaptomethyl)benzoyl |
| MMPA: 4-(mercaptomethyl)phenoxyacetyl |
| AMPA: 4-(aminomethyl)phenoxyacetyl |
| DMPPA: 4-(3,3-dimethyl-3-hydroxypropyl)phenoxyacetyl |
| Rink Amide: 2-(4-(amino(2,4-dimethoxyphenyl)methyl)phenoxy)acetyl |
| Xanthenyl: 4-((9-amino-9*H*-xanthen-3-yl)oxy)butanoyl |
| TCA: 5-(5-amino-10,11-dihydro-5*H*-dibenzo[*a*,*d*][7]annulen-3-yl)pentanoyl |
| 2-Chlorotrityl: 3-(4-(chloro(2-chlorophenyl)(phenyl)methyl)phenyl)propanoyl |

An example of such reaction can be seen in **FIGS 3****,** **8****,** **9**. These resulting GAP molecules can then be attached to any given amino acid to facilitate C terminus protection (as in **FIG 3**), side-chain protection (as in **FIG 4**), or N-terminus protection (a similar reaction as that seen in **FIG 17**). In another non-limiting example, novel GAP molecules can be synthesized by reacting HOBnDpp or a derivative thereof with any given amino acid, followed by reacting with any given linker molecule at the carboxylic acid moiety as can be seen in **FIG 5** and **9****.** The resulting GAP molecule can be used for C terminus protection (as in **FIG 3**), side-chain protection (as in **FIG 4**), or N-terminus protection (similarly to **FIG 17**).

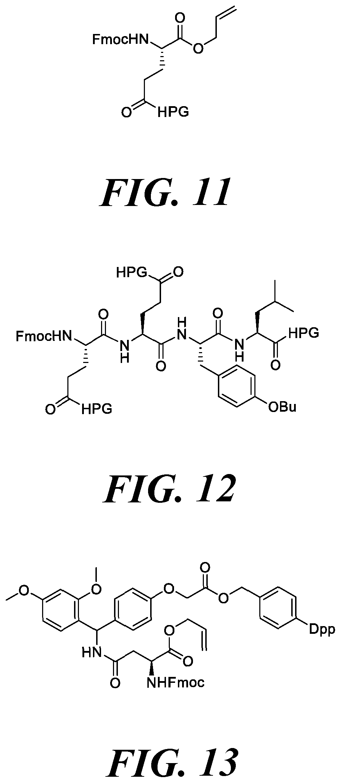

The synthesis strategy of the pharmacologically interesting, biologically active peptide bivalirudin is shown as a non-limiting preferred embodiment of the novel method of SolPPS herein disclosed. **FIG 6** depicts a schematic for attaching the novel GAP molecule HPG to the C-terminus of an unspecified amino acid, and this schematic is followed for the C-terminus protection of Fmoc-leucine-OH to form the compound shown in **FIG 10****.** In this particular embodiment, HPG is chosen as the C-terminus protecting group to allow for post-synthesis acid labile C-terminus deprotection without facilitating C-terminus amide formation. Following this initial step, standard Fmoc/tBu chemistry is run to couple Fmoc-Tyr(tBu)-OH and form the Fmoc-protected dipeptide. Subsequently, the schematic shown in **FIG 6** for amino acid side-chain protection is followed to protect the side chain of Fmoc-Glu-OAllyl with HPG before incorporation into the growing peptide, resulting in in the compound shown in **FIG 11****.** Allyl deprotection of the C-terminus of the Fmoc-Glu(HPG)-OAllyl is performed by treatment with Pd(PPh₃)₄ and triisopropyl or phenyl silane (see Isidro-Llobet 2009), followed by activation of the resulting molecule and coupling to the peptide. This process is repeated once more to attach the next HPG-protected glutamic acid residue, yielding the Fmoc-protected tetra-peptide shown in **FIG 12****.** Standard Fmoc/tBu peptide synthesis is performed to add proline and isoleucine, the above discussed procedure is performed to add the next two glutamic acid residues, and phenylalanine is subsequently added. The aspartic acid residue is protected with HPG and added just as glutamic acid following the schematic shown in **FIG 6****,** and the glycine residue is added thereafter.

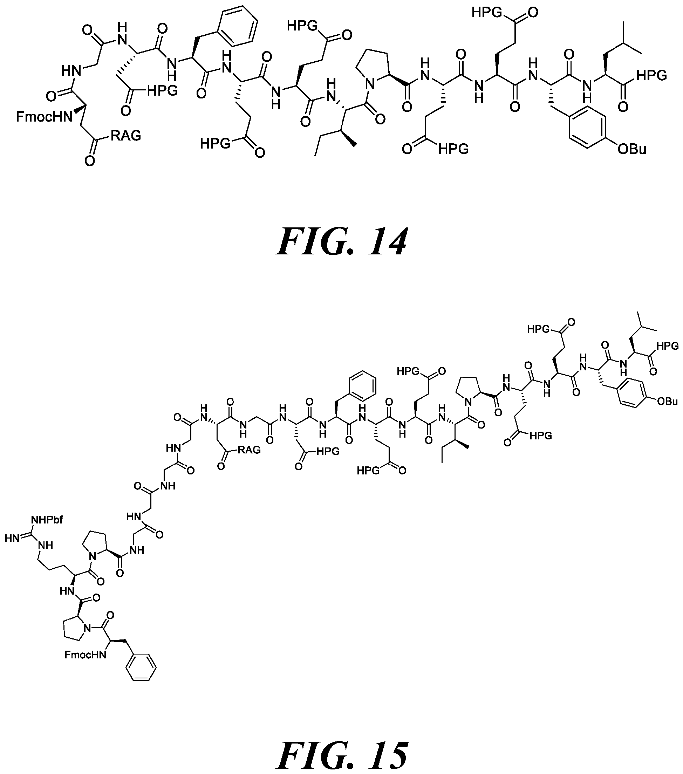

In another preferred embodiment, the novel GAP molecule RAG is reacted with Fmoc-Asp-OAllyl to yield Fmoc-Asp(RAG)-OAllyl, the compound shown in **FIG 13****.** Allyl deprotection of that compound is performed and it is subsequently activated and coupled to the peptide chain, yielding the Fmoc-protected peptide shown in **FIG 14****.** Subsequently, standard Fmoc/tBu peptide synthesis is carried out to add four glycines, proline, arginine, another proline, and D-phenylalanine to attain the fully protected bivalirudin peptide as shown in **FIG 15****.**

In this particular embodiment, after Fmoc-deprotection of the fully protected bivalirudin, a one-step global deprotection with a TFA cocktail (i.e. 90% TFA, 5% H₂O, 5% triisopropylsilane (TIPS)) can be performed to cleave all of the side chain protecting groups (including the novel GAP molecules), as well as the HPG C-terminus protecting group (see **FIG 16**). During this global deprotection, RAG-protected aspartic acid is also converted into deprotected asparagine. The molecule resulting from the global deprotection with the TFA cocktail is native bivalirudin.

**General procedure for Fmoc deprotection and coupling:** For the novel SolPPS method run in propylene carbonate (PC): To Fmoc-(AA)n-OBnDpp, Fmoc-(AA)n-HPG, Fmoc-(AA)n-RAG, or any amino acid protected at the C-terminus with any GAP protecting group, pre-dissolved in propylene carbonate (200 mM) is added deprotection base and octane thiol, followed by stirring at room temperature for 15 minutes with an alkane bilayer. Reaction mixture is then washed 2 times (X2) with fresh alkane solvent, and then washed 3 times with saturated ammonium chloride aqueous solution, and then dried. In a separate propylene carbonate solution is added 3.0 equivalents (eq.) of TBTU or TFFH, 3.0 eq Fmoc-AA-OH, and 3.0 eq. diisopropylethylamine (DIPEA) and the reaction is stirred for 7 min. This solution is then added to the previously dried PC solution containing H-(AA)n-(GAP molecule) and allowed to couple with stirring for 10-60 min. An excess of quenching agent is then added, such as long-chain (having 10 to 18 carbons linearly linked, i.e. C10-C18) aliphatic thiols, long-chain (C10-C18) aliphatic alcohols, long-chain (C10-C18) aliphatic amines, long-chain (C10-C18) aliphatic selenols, aliphatic polyamines, or aliphatic polyalcohols. The reaction mixture is then washed 3 times with saturated ammonium chloride aqueous solution and then dried to afford the elongated peptide in PC solution. This process is repeated as necessary to generate the peptide with the desired sequence and length. For the novel SolPPS method run in dichloromethane or ethyl acetate: To Fmoc-(AA)n-OBnDpp, Fmoc-(AA)n-HPG, Fmoc-(AA)n-RAG, or any amino acid protected at the C-terminus with any GAP protecting group, pre-dissolved in DCM or ethyl acetate (100 mM) is added deprotection base, followed by stirring at room temperature for 10 minutes. Reaction mixture is then washed 3 times with saturated ammonium chloride aqueous solution, 3 times with saturated sodium bicarbonate aqueous solution, and then dried. In a separate DCM or ethyl acetate solution is added 2.0 eq of TBTU or TFFH, 2.0 eq Fmoc-AA-OH, and 5.0 eq. DIPEA and the reaction is stirred for 7 min. This solution is then added to the previously dried DCM or ethyl acetate solution containing H-(AA)n-(GAP molecule) and allowed to couple with stirring for 10-60 min. An excess of quenching agent is then added, such as long-chain (C10-C18) aliphatic thiols, long-chain (C10-C18) aliphatic alcohols, long-chain (C10-C18) aliphatic amines, long-chain (C10-C18) aliphatic selenols, aliphatic polyamines, or aliphatic polyalcohols. The reaction mixture is then washed 3 times with saturated ammonium chloride aqueous solution and then dried to afford the elongated peptide in DCM or ethyl acetate solution. This process is repeated as needed to generate the peptide with the desired sequence and length.

**General procedure for EDCI coupling:** Reaction mixture containing a deprotected N-terminus of an amino acid or peptide is cooled in an ice bath. 2 eq of the amino acid to be coupled to such free N-terminus is subsequently added to the reaction mixture, followed by 2 eq of EDCI. The resulting mixture is removed from the ice bath and stirred for 1 hour, and an excess of quenching agent is then added, such as long-chain (C10-C18) aliphatic thiols, long-chain (C10-C18) aliphatic alcohols, long-chain (C10-C18) aliphatic amines, long-chain (C10-C18) aliphatic selenols, aliphatic polyamines, or aliphatic polyalcohols. Reaction mixture is then washed 3 times with saturated ammonium chloride aqueous solution, 3 times with saturated sodium bicarbonate aqueous solution, and then dried.

Functionality may also be, in whole or in part, distributed among multiple components, in manners now known or to become known. Thus, myriad combinations are possible in achieving the functions, features, and preferences described herein. Moreover, the scope of the present disclosure covers conventionally known manners for carrying out the described features as well as those variations and modifications that may be made to the processes, composition, or compounds described herein as would be understood by those skilled in the art now and hereafter.

Furthermore, the embodiments of methods presented and described as diagrams, schematics or flowcharts in this disclosure (such as the Figures) are provided by way of example in order to provide a more complete understanding of the technology. The disclosed methods are not limited to the operations and logical flow presented herein. Alternative embodiments are contemplated in which the order of the various operations is altered and in which sub-operations described as being part of a larger operation are performed independently. While various embodiments have been described for purposes of this disclosure, such embodiments should not be deemed to limit the teaching of this disclosure to those embodiments. Various changes and modifications may be made to the elements and operations described above to obtain a result that remains within the scope of the systems and processes described in this disclosure.

### RELATED REFERENCES

The below references are given herein as examples.
An, G.; Seifert, C.; Li, G. N-Phosphonyl/phosphinyl imines and group- assisted purification (GAP) chemistry/technology. Org. Biomol. Chem. 2015, 13, 1600-1617.
Ai, T.; Li, G. Chiral N-phosphonyl imine chemistry: Asymmetric synthesis of a,β-diamino esters by reacting phosphonyl imines with glycine enolates. Bioorg. Med. Chem. Lett. 2009, 19, 3967-3969.
Han, J.; Ai, T.; Nguyen, T.; Li, G. Chiral N-phosphonyl imine chemistry: asymmetric additions of ester enolates for the synthesis of β-amino acids. Chem. Biol. Drug Des. 2008, 72, 120-126.
Kattamuri, P. V.; Ai, T.; Pindi, S.; Sun, Y.; Gu, P.; Shi, M.; Li, G. Asymmetric Synthesis of α-Amino-1,3-dithianes via Chiral N-Phosphonyl Imine-Based Umpolung Reaction Without Using Chromatography and Recrystallization. J. Org. Chem. 2011, 76, 2792-2797.
Kattuboina, A.; Kaur, P.; Nguyen, T.; Li, G. Chiral N-phosphonyl imine chemistry: asymmetric 1,2-additions of allylmagnesium bromides. Tetrahedron Lett. 2008, 49, 3722-3724.
Kattuboina, A.; Li, G. Chiral N-phosphonyl imine chemistry: new reagents and their applications for asymmetric reactions. Tetrahedron Lett. 2008, 49, 1573-1577.
Kaur, P.; Wever, W.; Pindi, S.; Milles, R.; Gu, P.; Shi, M.; Li, G. The GAP chemistry for chiral N-phosphonyl imine-based Strecker reaction. Green Chem. 2011, 13, 1288-1292.
Pindi, S.; Kaur, P.; Shakya, G.; Li, G. N-Phosphinyl Imine Chemistry (I): Design and Synthesis of Novel N-Phosphinyl Imines and their Application to Asymmetric aza-Henry Reaction. Chem. Biol. Drug. Des. 2011, 77, 20-29.
Xie, J.-b.; Luo, J.; Winn, T. R.; Cordes, D. B.; Li, G. Group-assisted purification (GAP) chemistry for the synthesis of Velcade via asymmetric borylation of Nphosphinylimines. Beilstein J. Org. Chem. 2014, 10, 746-751.
Dailler, D.; Danoun, G.; Baudoin, O. A General and Scalable Synthesis of
Aeruginosin Marine Natural Products Based on Two Strategic C(sp(3))-H Activation
Reactions. Angewandte Chemie-International Edition 2015, 54, 4919-4922.
Kaufmann, E.; Hattori, H.; Miyatake-Ondozabal, H.; Gademann, K. Total Synthesis of the Glycosylated Macrolide Antibiotic Fidaxomicin. Organic Letters 2015, 17, 3514-3517.
Sharma, P. K.; Romanczyk, L. J.; Kondaveti, L.; Reddy, B.; Arumugasamy, J.; Lombardy, R.; Gou, Y.; Schroeter, H. Total Synthesis of Proanthocyanidin A1, A2, and Their Stereoisomers. Organic Letters 2015, 17, 2306-2309.
Wuts, P. G. M. Greene's Protective Groups in Organic Synthesis. 5 ed.; John Wiley & Sons, Inc: New Jersey, 2014.
Isidro-Llobet, A.; Alvarez, M.; Albericio, F. Amino Acid-Protecting Groups. Chem. Rev. 2009, 109, 2455-2504.
Behrendt, R.; Huber, S.; Marti, R.; White, P. New t-butyl based aspartate protecting groups preventing aspartimide formation in Fmoc SPPS. Journal of Peptide Science 2015, 21, 680-687.
Chandrudu, S.; Simerska, P.; Toth, I. Chemical Methods for Peptide and Protein Production. Molecules 2013, 18, 4373.
Mochizuki, M.; Tsuda, S.; Tanimura, K.; Nishiuchi, Y. Regioselective Formation of Multiple Disulfide Bonds with the Aid of Postsynthetic S-Tritylation. Organic Letters 2015, 17, 2202-2205.
Merrifield, R. B. Solid Phase Peptide Synthesis. I. The Synthesis of a Tetrapeptide. J. Am. Chem. Soc. 1963, 85, 2149.
Mollica, A.; Pinnen, F.; Azzurra, S.; Costante, R. The Evolution of Peptide Synthesis: From Early Days to Small Molecular Machines. Curr. Bioact. Compd. 2013, 9, 184-202.
Shelton, P. T.; Jensen, K. J. Linkers, Resins, and General Procedures for Solid-Phase Peptide Synthesis. In Peptide Synthesis and Applications, 2nd Edition, Jensen, K. J.; Shelton, P. T.; Pedersen, S. L., Eds. Humana Press Inc: Totowa, 2013; Vol. 1047, pp 23-41.
An, G.; Seifert, C.; Sun, H.; Pan, Y.; Li, G. Group-Assisted Purification (GAP) for Protection of Amino Acids Using N-Phosphonyl Functional Groups. Heterocycles 2015, 90, 344-356.
An, G.; Zhou, W.; Xu, X.; Pan, Y.; Li, G. Solution-Phase-Peptide Synthesis Without Purification of Column Chromatography and Recrystallization by Protecting Amino Acid Esters with Phosphinyl Chloride. Heterocycles 2015, 90, 1405-1418.
Wu, J.; An, G.; Lin, S.; Xie, J.; Zhou, W.; Sun, H.; Pan, Y.; Li, G. Solution phase peptide synthesis via the group-assisted purification (GAP) chemistry without using chromatography and recrystallization. Chem. Commun. 2014, 50, 1259-1261.
Brieke, C.; Cryle, M. J. A Facile Fmoc Solid Phase Synthesis Strategy To Access Epimerization-Prone Biosynthetic Intermediates of Glycopeptide Antibiotics. Organic Letters 2014, 16, 2454-2457.
Chen, C.-C.; Rajagopal, B.; Liu, X. Y.; Chen, K. L.; Tyan, Y.-C.; Lin, F.; Lin, P.-C. A mild removal of Fmoc group using sodium azide. Amino Acids 2014, 46, 367-374.
Spinella, M.; De Marco, R.; Belsito, E. L.; Leggio, A.; Liguori, A. The dimethylsulfoxonium methylide as unique reagent for the simultaneous deprotection of amino and carboxyl function of N-Fmoc-α-amino acid and N-Fmoc-peptide esters. Tetrahedron 2013, 69, 2010-2016.
Amblard, M.; Enomoto, H.; Subra, G.; Fehrentz, J.-A.; Martinez, J. The Fundamentals of Fmoc Solid-Phase Peptide Synthesis. Idenshi Igaku Mook 2012, 21, 36-42.
Shi, M.; Yang, Y.; Zhou, X.; Cai, L.; Fang, C.; Wang, C.; Sun, H.; Sun, Y.; Gao, Y.; Gu, J.; Fawcett, J. P. Determination of thymopentin in beagle dog blood by liquid chromatography with tandem mass spectrometry and its application to a preclinical pharmacokinetic study. Journal of Separation Science 2015, 38, 1351-1357.
Zhu, M.-X.; Wan, W.-L.; Li, H.-S.; Wang, J.; Chen, G.-A.; Ke, X.-Y. Thymopentin enhances the generation of T-cell lineage derived from human embryonic stem cells in vitro. Experimental Cell Research 2015, 331, 387-398.
Fu, T. T.; Qiao, H. W.; Peng, Z. M.; Hu, G. B.; Wu, X. J.; Gao, Y. X.; Zhao,
Y. F. Palladium-catalyzed air-based oxidative coupling of arylboronic acids with H-phosphine oxides leading to aryl phosphine oxides. Organic & Biomolecular Chemistry 2014, 12, 2895-2902.
Lawrenson, S.B.; Arav, R.; North, M.. The greening of peptide synthesis. Green Chemistry 2017, 19, 1685.
Isidro-Llobet, A.; Alvarez, M.; Albericio, F. Amino Acid-Protecting Groups. Chemical Reviews 2009, 109, 2455 - 2504.
Jensen, Knud J. Chapter 1: Peptide Synthesis. Pharmaceutical Formulation Development of Peptides and Proteins 2013, pages 1-16.
Amblard, M.; Fehrentz, J.A.; Martinez, J.; Subra, G. Methods and Protocols of Modern Solid Phase Peptide Synthesis. Molecular Biotechnology 2006, 33, 239-254.
Bachem. Tips and Trick for Solid Phase Peptide Synthesis from the Experts at Bachem. Solid Phase Peptide Synthesis 2016, pages 1-55.

## Claims

1. A protecting group for solution-phase peptide synthesis, the protecting group having a chemical formula selected from the group consisting of Chemical Formula 1, Chemical Formula 2, Chemical Formula 3, Chemical Formula 4, Chemical Formula 5, Chemical Formula 6, Chemical Formula 7, and Chemical Formula 8;
wherein Chemical Formula 1 is: 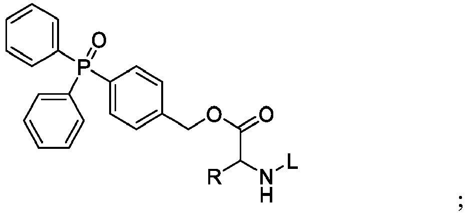
wherein Chemical Formula 2 is: 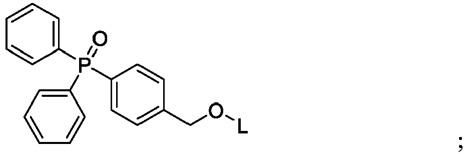
wherein Chemical Formula 3 is: 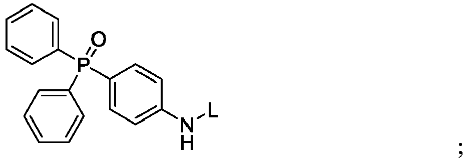
wherein Chemical Formula 4 is: 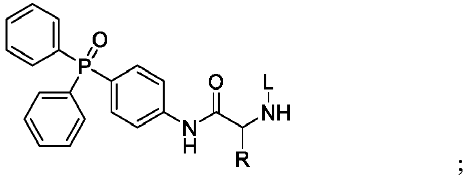
wherein Chemical Formula 5 is: 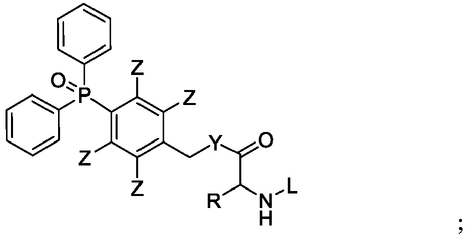
wherein Chemical Formula 6 is: 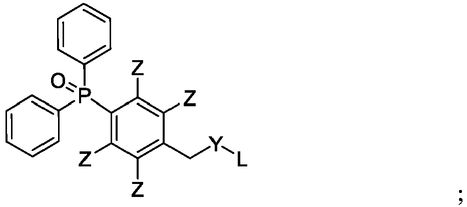
wherein Chemical Formula 7 is: 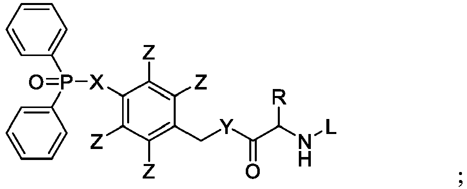
wherein Chemical Formula 8 is: 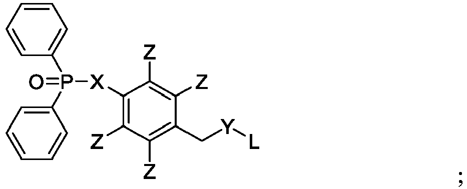 wherein:
Z is selected from the group consisting of: -H, a methyl group (-Me), and a methoxy group (-OMe);
Y is selected from the group consisting of: -O-, -S-, and -NH-;
X is selected from the group consisting of: -O-, -S-, and -NH-;
R is selected from the group consisting ofH, methyl, -CH₂SH, -CH₂CH₂COOH, - CH₂COOH, benzyl, 4-(1H-imidazolyl)methyl, -CH(CH₃)(CH₂CH₃), - CH₂CH₂CH₂CH₂NH₂, -CH₂CH₂CH₂NH₂, isobutyl, -CH₂CH₂SCH₃, -CH₂CONH₂, - CH₂CH₂CONH₂, a propyl group wherein the 1-position of the propyl group is attached to the same position as the R group and the 3-position of the propyl group is attached to the nitrogen, -CH₂CH₂CH₂N=C(NH₂)₂, -CH₂OH, 1-hydroxyethyl, isopropyl, 4-hydroxybenzyl, and 3-(1H-indolyl)methyl; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

2. The protecting group of claim 1, wherein the protecting group is selected from a group consisting of Chemical Formula 1, Chemical Formula 4, Chemical Formula 5, and Chemical Formula 7; and wherein R is the side-chain of an amino acid.

3. A method of performing solution-phase peptide synthesis, wherein the method comprises the steps of:
attaching a first protecting group to a first amino acid to provide a first protected amino acid, wherein the first protecting group is the protecting group of any of the preceding claims; and
performing a coupling reaction on the first protected amino acid or a peptide formed by linking the first amino acid to another molecule.

4. The method of claim 3, wherein the first protecting group is attached to the C-terminus of the first amino acid; and/or
wherein the first protecting group is attached to the side-chain of the first amino acid; and/or
wherein the first protecting group is attached to the N-terminus of the first amino acid.

5. The method of claim 3 or 4, wherein the method comprises:
coupling a second protected amino acid to the first protected amino acid;
wherein the second protected amino acid comprises a second protecting group and a second amino acid, wherein the second amino acid comprises a side chain, wherein the second protecting group is attached to the side chain of the second amino acid; wherein the second protecting group has a chemical formula selected from the group consisting of Chemical Formula 1, Chemical Formula 2, Chemical Formula 3, Chemical Formula 4, Chemical Formula 5, Chemical Formula 6, Chemical Formula 7, and Chemical Formula 8.

6. The method of any of the claims 3 to 5, wherein the step of attaching the first protecting group comprises attaching the first protecting group to the C-terminus of the first amino acid, the N-terminus of the first amino acid, or the side chain of the first amino acid.

7. A method of forming a protecting group for solution-phase peptide synthesis, the method comprising:
coupling benzyl diphenylphosphine oxide (HOBnDpp), or aniline diphenylphosphine oxide (NH₂PhDpp), (i) directly to a linker molecule, or (ii) to a linker molecule via an amino acid,;
wherein the coupling step comprises coupling 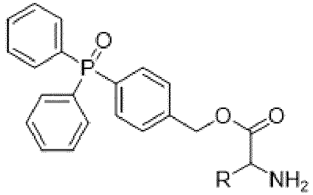
to L-OH to form the protecting group, wherein the protecting group is defined by Chemical Formula 1: 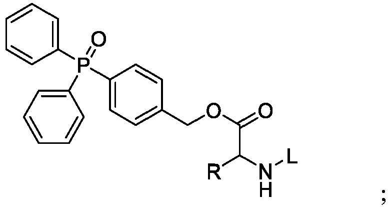 wherein:
R is selected from the group consisting of H, methyl, -CH₂SH, -CH₂CH₂COOH, - CH₂COOH, benzyl, 4-(1H-imidazolyl)methyl, -CH(CH₃)(CH₂CH₃), -CH₂CH₂CH₂CH₂NH₂, -CH₂CH₂CH₂NH₂, isobutyl, -CH₂CH₂SCH₃, -CH₂CONH₂, -CH₂CH₂CONH₂, a propyl group wherein the 1-position of the propyl group is attached to the same position as the R group and the 3-position of the propyl group is attached to the nitrogen, - CH₂CH₂CH₂N=C(NH₂)₂, -CH₂OH, 1-hydroxyethyl, isopropyl, 4-hydroxybenzyl, and 3-(1H-indolyl)methyl; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

8. The method of claim 7, wherein the coupling step comprises coupling 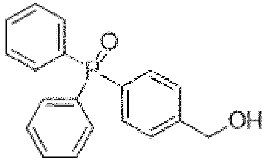 to L-OH to form the protecting group, wherein the protecting group is defined by Chemical Formula 2: 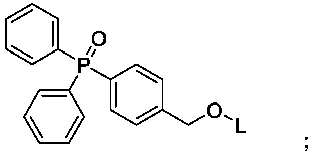 wherein:
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

9. The method of any of the claims 7 or 8, wherein the coupling step comprises coupling 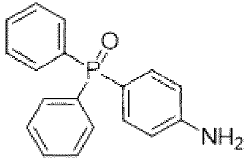 to L-OH to form the protecting group, wherein the protecting group is defined by Chemical Formula 3: 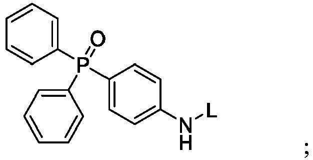 wherein:
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

10. The method of any of the claims 7 to 9, wherein the coupling step comprises coupling 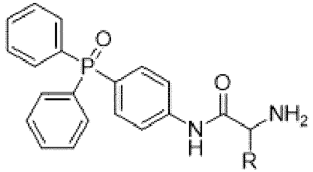 to L-OH to form the protecting group, wherein the protecting group is defined by Chemical Formula 4: 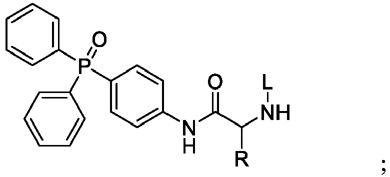 wherein:
R is selected from the group consisting of known side chains of protected amino acids and known side chains of unprotected amino acids; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

11. The method ofany of the claims 7 to 10, wherein the coupling step comprises coupling 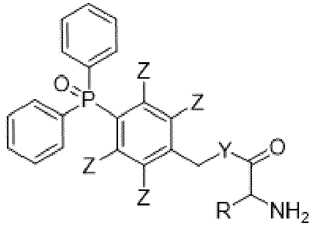 to L-OH to form the protecting group, wherein the protecting group is defined by Chemical Formula 5: 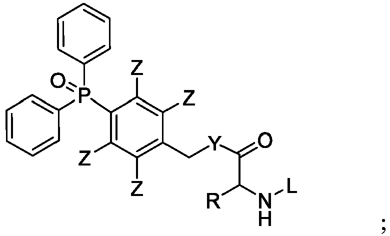 wherein:
Z is selected from the group consisting of: -H, -Me, and -OMe;
Y is selected from the group consisting of: -O-, -S-, and -NH-;
R is selected from the group consisting of known side chains of protected amino acids and known side chains of unprotected amino acids; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

12. The method of any of the claims 7 to 11, wherein the coupling step comprises coupling 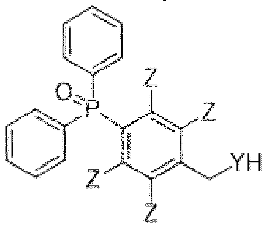 to L-OH to form the protecting group, wherein the protecting group is defined by Chemical Formula 6: 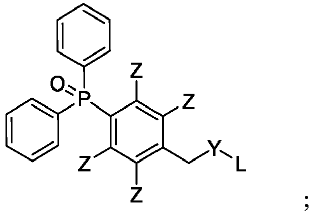 wherein:
Z is selected from the group consisting of: -H, -Me, and -OMe;
Y is selected from the group consisting of: -O-, -S-, and -NH-; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

13. The method of any of the claims 7 to 12, wherein the coupling step comprises coupling 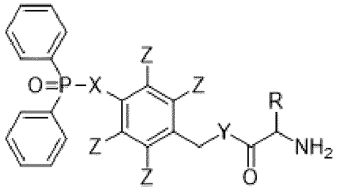 to L-OH to form the protecting group, wherein the protecting group is defined by Chemical Formula 7: 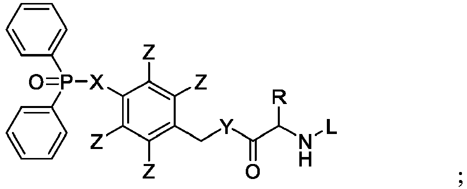 wherein:
Z is selected from the group consisting of: -H, -Me, and -OMe;
Y is selected from the group consisting of: -O-, -S-, and -NH-;
X is selected from the group consisting of: -O-, -S-, and -NH-;
R is selected from the group consisting of known side chains of protected amino acids and known side chains of unprotected amino acids; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl"); and/or
wherein the coupling step comprises coupling 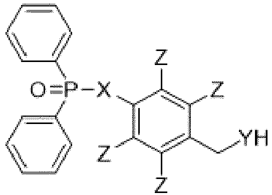
to L-OH to form the protecting group, wherein the protecting group is defined by Chemical Formula 8: 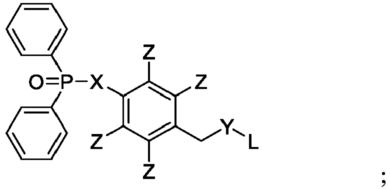 wherein:
Z is selected from the group consisting of: -H, -Me, and -OMe;
Y is selected from the group consisting of: -O-, -S-, and -NH-;
X is selected from the group consisting of: -O-, -S-, and -NH-; and
L is selected from the group consisting of: 4-(hydroxymethyl)phenoxyacetyl ("HMPA"), 4-(hydroxymethyl)phenoxybutanoyl ("HMPB"), 4-(hydroxymethyl)benzoyl ("HMB"), 4-(mercaptomethyl)benzoyl ("MMB"), 4-(mercaptomethyl)phenoxyacetyl ("MMPA"), 4-(aminomethyl)phenoxyacetyl ("AMPA"), 4-(3,3-dimethyl-3-hydroxypropyl)phenoxyacetyl ("DMPPA"), 2-(4-(amino(2,4-dimethoxyphenyl)methyl)phenoxy)acetyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA"), and 3-(4-(chloro(2-chlorophenyl)(phenyl)methyl)phenyl)propanoyl ("2-Chlorotrityl").

## Patentansprüche

1. Schutzgruppe für Peptidsynthese in der Lösungsphase, wobei die Schutzgruppe eine chemische Formel aufweist, die aus der Gruppe bestehend aus der chemischen Formel 1, der chemischen Formel 2, chemischen Formel 3, der chemischen Formel 4, der chemischen Formel 5, der chemischen Formel 6 und der chemischen Formel 7 und der chemischen Formel 8 ausgewählt ist;
wobei die chemische Formel 1 lautet: 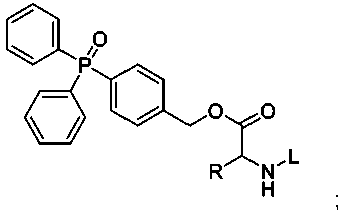
wobei die chemische Formel 2 lautet: 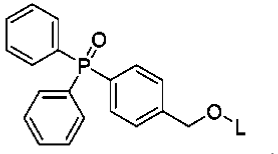
wobei die chemische Formel 3 lautet: 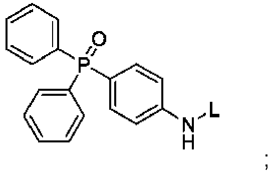
wobei die chemische Formel 4 lautet: 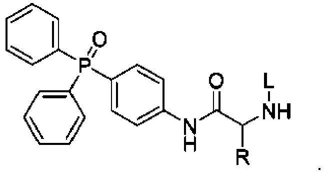
wobei die chemische Formel 5 lautet: 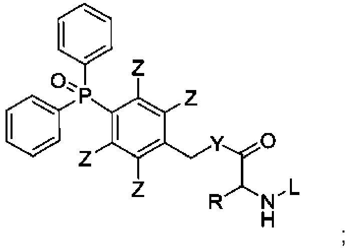
wobei die chemische Formel 6 lautet: 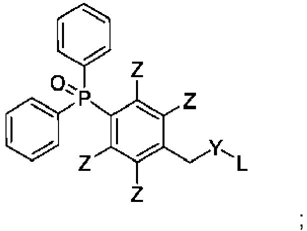
wobei die chemische Formel 7 lautet: 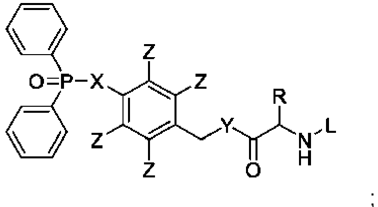
wobei die chemische Formel 8 lautet: 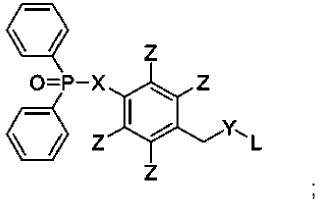 wobei:
Z ausgewählt ist aus der Gruppe bestehend aus: -H, einer Methylgruppe (-Me) und einer Methoxygruppe (-OMe);
Y ausgewählt ist aus der Gruppe bestehend aus: -O-, -S- und -NH-;
X ausgewählt ist aus der Gruppe bestehend aus: -O-, -S- und -NH-;
R ausgewählt ist aus der Gruppe bestehend aus H, Methyl, -CH₂SH, -CH₂CH₂COOH, -CH₂COOH, Benzyl, 4-(1H-Imidazolyl)methyl, -CH(CH₃)(CH₂CH₃), -CH₂CH₂CH₂CH₂NH₂, -CH₂CH₂CH₂NH₂, Isobutyl, -CH₂CH₂SCH₃, -CH₂CONH₂, -CH₂CH₂CONH₂, einer Propylgruppe, wobei die 1-Position der Propylgruppe an die gleiche Position wie die R-Gruppe gebunden ist und die 3-Position der Propylgruppe an den Stickstoff gebunden ist, -CH₂CH₂CH₂N=C(NH₂)₂, -CH₂OH, 1-Hydroxyethyl, Isopropyl, 4-Hydroxybenzyl und 3-(1H-Indolyl)methyl; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11n-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

2. Schutzgruppe nach Anspruch 1, wobei die Schutzgruppe aus einer Gruppe bestehend aus der chemischen Formel 1, der chemischen Formel 4, der chemischen Formel 5 und der chemischen Formel 7 ausgewählt ist; und wobei R die Seitenkette einer Aminosäure ist.

3. Verfahren zur Durchführung einer Peptidsynthese in der Lösungsphase, wobei das Verfahren die folgenden Schritte umfasst:
Binden einer ersten Schutzgruppe an eine erste Aminosäure, um eine erste geschützte Aminosäure bereitzustellen, wobei die erste Schutzgruppe die Schutzgruppe nach einem der vorhergehenden Ansprüche ist; und
Durchführen einer Kopplungsreaktion an der ersten geschützten Aminosäure oder einem Peptid, das durch Koppeln der ersten Aminosäure an ein anderes Molekül gebildet wird.

4. Verfahren nach Anspruch 3, wobei die erste Schutzgruppe an den C-Terminus der ersten Aminosäure gebunden wird; und/oder
wobei die erste Schutzgruppe an die Seitenkette der ersten Aminosäure gebunden wird; und/oder
wobei die erste Schutzgruppe an den N-Terminus der ersten Aminosäure gebunden wird.

5. Verfahren nach Anspruch 3 oder 4, wobei das Verfahren umfasst:
Koppeln einer zweiten geschützten Aminosäure an die erste geschützte Aminosäure;
wobei die zweite geschützte Aminosäure eine zweite Schutzgruppe und eine zweite Aminosäure umfasst, wobei die zweite Aminosäure eine Seitenkette umfasst, wobei die zweite Schutzgruppe an die Seitenkette der zweiten Aminosäure gebunden wird; wobei die zweite Schutzgruppe eine chemische Formel aufweist, die ausgewählt ist aus der Gruppe bestehend aus der chemischen Formel 1, der chemischen Formel 2, chemischen Formel 3, der chemischen Formel 4, der chemischen Formel 5, der chemischen Formel 6, der chemischen Formel 7 und der chemischen Formel 8.

6. Verfahren nach einem der Ansprüche 3 bis 5, wobei der Schritt des Bindens der ersten Schutzgruppe ein Binden der ersten Schutzgruppe an den C-Terminus der ersten Aminosäure, den N-Terminus der ersten Aminosäure oder die Seitenkette der ersten Aminosäure umfasst.

7. Verfahren zur Bildung einer Schutzgruppe für die Peptidsynthese in der Lösungsphase, wobei das Verfahren umfasst:
Koppeln von Benzyldiphenylphosphinoxid (HOBnDpp) oder Anilindiphenylphosphinoxid (NH₂PhDpp) (i) direkt an ein Linkermolekül oder (ii) über eine Aminosäure an ein Linkermolekül;
wobei der Kopplungsschritt das Koppeln von 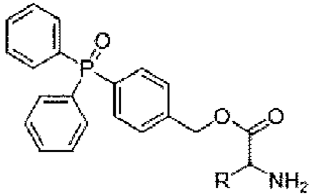
an L-OH umfasst, um die Schutzgruppe zu bilden, wobei die Schutzgruppe durch die folgende chemische Formel 1 definiert ist: 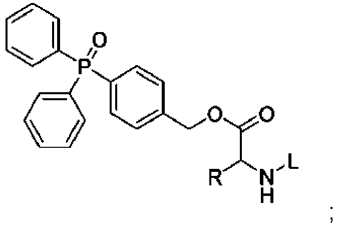 wobei:
R ausgewählt ist aus der Gruppe bestehend aus H, Methyl, -CH₂SH, -CH₂CH₂COOH, -CH₂COOH, Benzyl, 4-(1H-Imidazolyl)methyl, -CH(CH₃)(CH₂CH₃), -CH₂CH₂CH₂CH₂NH₂, -CH₂CH₂CH₂NH₂, Isobutyl, -CH₂CH₂SCH₃, -CH₂CONH₂, -CH₂CH₂CONH₂, einer Propylgruppe, wobei die 1-Position der Propylgruppe an die gleiche Position wie die R-Gruppe gebunden ist und die 3-Position der Propylgruppe an den Stickstoff gebunden ist, -CH₂CH₂CH₂N=C(NH₂)₂, -CH₂OH, 1-Hydroxyethyl, Isopropyl, 4-Hydroxybenzyl und 3-(1H-Indolyl)methyl; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

8. Verfahren nach Anspruch 7, wobei der Kopplungsschritt das Koppeln von 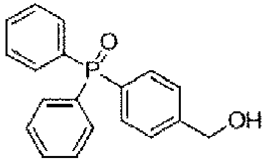 an L-OH umfasst, um die Schutzgruppe zu bilden, wobei die Schutzgruppe durch die folgende chemische Formel 2 definiert ist: 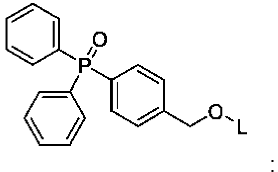 wobei:
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

9. Verfahren nach einem der Ansprüche 7 oder 8, wobei der Kopplungsschritt das Koppeln von 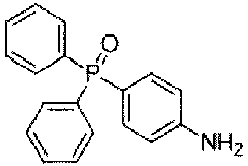 an L-OH umfasst, um die Schutzgruppe zu bilden, wobei die Schutzgruppe durch die folgende chemische Formel 3 definiert ist: 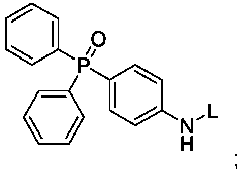 wobei:
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

10. Verfahren nach einem der Ansprüche 7 bis 9, wobei der Kopplungsschritt das Koppeln von 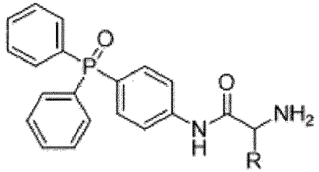 an L-OH umfasst, um die Schutzgruppe zu bilden, wobei die Schutzgruppe durch die folgende chemische Formel 4 definiert ist: 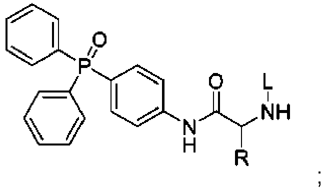 wobei:
R ausgewählt ist aus der Gruppe bestehend aus bekannten Seitenketten geschützter Aminosäuren und bekannten Seitenketten ungeschützter Aminosäuren; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

11. Verfahren nach einem der Ansprüche 7 bis 10, wobei der Kopplungsschritt das Koppeln von 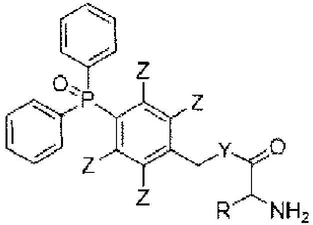 an L-OH umfasst, um die Schutzgruppe zu bilden, wobei die Schutzgruppe durch die folgende chemische Formel 5 definiert ist: 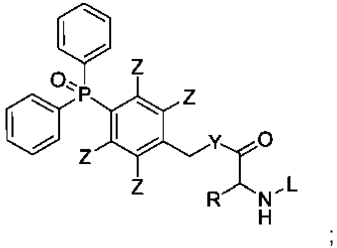 wobei:
Z ausgewählt ist aus der Gruppe bestehend aus: -H, -Me und -OMe;
Y ausgewählt ist aus der Gruppe bestehend aus: -O-, -S- und -NH-;
R ausgewählt ist aus der Gruppe bestehend aus bekannten Seitenketten geschützter Aminosäuren und bekannten Seitenketten ungeschützter Aminosäuren; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

12. Verfahren nach einem der Ansprüche 7 bis 11, wobei der Kopplungsschritt das Koppeln von 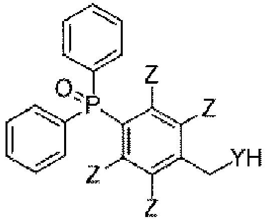 an L-OH umfasst, um die Schutzgruppe zu bilden, wobei die Schutzgruppe durch die folgende chemische Formel 6 definiert ist: 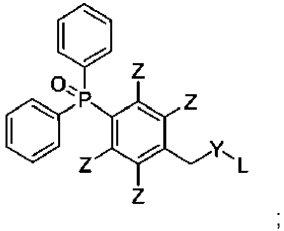 wobei:
Z ausgewählt ist aus der Gruppe bestehend aus: -H, -Me und -OMe;
Y ausgewählt ist aus der Gruppe bestehend aus: -O-, -S- und -NH-; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

13. Verfahren nach einem der Ansprüche 7 bis 12, wobei der Kopplungsschritt das Koppeln von 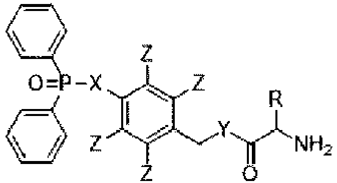 an L-OH umfasst, um die Schutzgruppe zu bilden, wobei die Schutzgruppe durch die folgende chemische Formel 7 definiert ist: 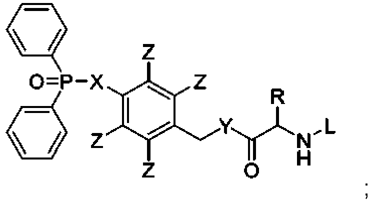 wobei:
Z ausgewählt ist aus der Gruppe bestehend aus: -H, -Me und -OMe;
Y ausgewählt ist aus der Gruppe bestehend aus: -O-, -S- und -NH-;
X ausgewählt ist aus der Gruppe bestehend aus: -O-, -S- und -NH-;
R ausgewählt ist aus der Gruppe bestehend aus bekannten Seitenketten geschützter Aminosäuren und bekannten Seitenketten ungeschützter Aminosäuren; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl"); und/oder
wobei der Kopplungsschritt das Koppeln von 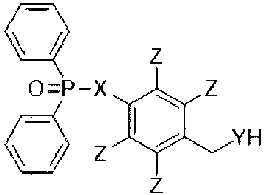
an L-OH umfasst, um die Schutzgruppe zu bilden, wobei die Schutzgruppe durch die folgende chemische Formel 8 definiert ist: 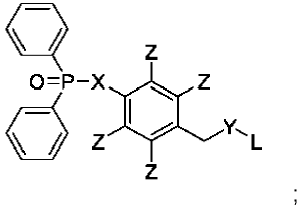 wobei:
Z ausgewählt ist aus der Gruppe bestehend aus: -H, -Me und -OMe;
Y ausgewählt ist aus der Gruppe bestehend aus: -O-, -S- und -NH-;
X ausgewählt ist aus der Gruppe bestehend aus: -O-, -S- und -NH-; und
L ausgewählt ist aus der Gruppe bestehend aus: 4-(Hydroxymethyl)phenoxyacetyl ("HMPA"), 4-(Hydroxymethyl)phenoxybutanoyl ("HMPB"), 4-(Hydroxymethyl)benzoyl ("HMB"), 4-(Mercaptomethyl)benzoyl ("MMB"), 4-(Mercaptomethyl)phenoxyacetyl ("MMPA"), 4-(Aminomethyl)phenoxyacetyl ("AMPA"), 4-(3,3-Dimethyl-3-hydroxypropyl)phenoxyacetyl ("DMPPA"), 2-(4-(Amino(2,4-dimethoxyphenyl)methyl)phenoxy)acetyl ("Rink Amide"), 4-((9-Amino-9H-xanthen-3-yl)oxy)butanoyl ("Xanthenyl"), 5-(5-Amino-10,11-dihydro-5H-dibenzo[a,d][7]annulen-3-yl)pentanoyl ("TCA") und 3-(4-(Chlor(2-chlorphenyl)(phenyl)methyl)phenyl)propanoyl ("2-Chlortrityl").

## Revendications

1. Groupe protecteur pour une synthèse peptidique en phase solution, le groupe protecteur ayant une formule chimique choisie dans le groupe composé de la formule chimique 1, la formule chimique 2, la formule chimique 3, la formule chimique 4, la formule chimique 5, la formule chimique 6, la formule chimique 7 et formule chimique 8 ;
où la formule chimique 1 est : 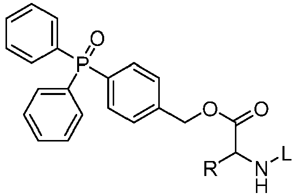
où la formule chimique 2 est : 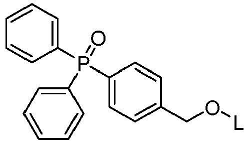
où la formule chimique 3 est : 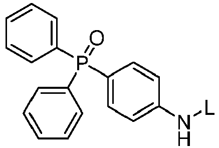
où la formule chimique 4 est : 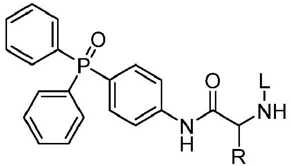
où la formule chimique 5 est : 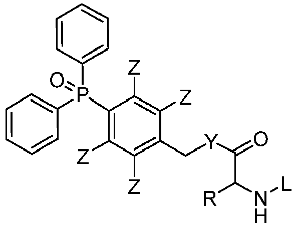
où la formule chimique 6 est : 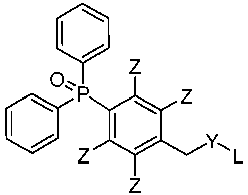
où la formule chimique 7 est : 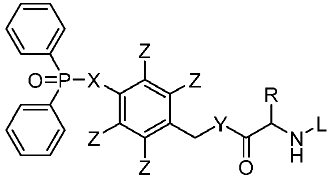
où la formule chimique 8 est : 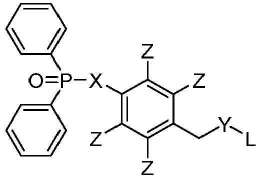 où :
Z est choisi dans le groupe composé de : -H, un groupe méthyle (-Me) et un groupe méthoxy (-OMe) ;
Y est choisi dans le groupe composé de : -O-, -S- et -NH- ;
X est choisi dans le groupe compose de : -O-, -S- et -NH- ;
R est choisi dans le groupe composé de H, méthyle, -CH₂SH, -CH₂CH₂COOH, - CH₂COOH, benzyle, 4-(1H-imidazolyl)méthyle, -CH(CH₃)(CH₂CH₃), - CH₂CH₂CH₂CH₂NH₂, -CH₂CH₂CH₂NH₂, isobutyle, -CH₂CH₂SCH₃, -CH₂CONH₂, - CH₂CH₂CONH₂, un groupe propyle dans lequel la position 1 du groupe propyle est attachée à la même position que le groupe R et la position 3 du groupe propyle est attachée à l'azote, à -CH₂CH₂CH₂N=C(NH₂)₂, à -CH₂OH, à l'1-hydroxyéthyle, à l'isopropyle, au 4-hydroxybenzyle et au 3-(1H-indolyl)méthyle ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

2. Groupe protecteur selon la revendication 1, dans lequel le groupe protecteur est choisi dans le groupe composé de la formule chimique 1, de la formule chimique 4, de la formule chimique 5 et de la formule chimique 7, et dans lequel R est la chaîne latérale d'un acide aminé.

3. Procédé de synthèse peptidique en phase solution, le procédé comprenant les étapes suivantes :
attacher un premier groupe protecteur à un premier acide aminé pour fournir un premier acide aminé protégé, où le premier groupe protecteur est le groupe protecteur de l'une quelconque des revendications précédentes ; et
effectuer une réaction de couplage sur le premier acide aminé protégé ou un peptide formé en reliant le premier acide aminé à une autre molécule.

4. Procédé selon la revendication 3, dans lequel le premier groupe protecteur est attaché à l'extrémité C du premier acide aminé ; et/ou
dans lequel le premier groupe protecteur est attaché à la chaîne latérale du premier acide aminé ; et/ou
dans lequel le premier groupe protecteur est attaché à l'extrémité N du premier acide aminé.

5. Procédé selon la revendication 3 ou 4, le procédé comprenant :
le couplage d'un deuxième acide aminé protégé au premier acide aminé protégé ;
dans lequel le deuxième acide aminé protégé comprend un deuxième groupe protecteur et un deuxième acide aminé, dans lequel le deuxième acide aminé comprend une chaîne latérale, dans lequel le deuxième groupe protecteur est attaché à la chaîne latérale du deuxième acide aminé ; dans lequel le deuxième groupe protecteur a une formule chimique choisie dans le groupe composé de la formule chimique 1, la formule chimique 2, la formule chimique 3, la formule chimique 4, la formule chimique 5, la formule chimique 6, la formule chimique 7 et la formule chimique 8.

6. Procédé selon l'une quelconque des revendications 3 à 5, dans lequel l'étape consistant à attacher le premier groupe protecteur consiste à attacher le premier groupe protecteur à l'extrémité C du premier acide aminé, l'extrémité N du premier acide aminé ou la chaîne latérale du premier acide aminé.

7. Procédé de formation d'un groupe protecteur pour la synthèse peptidique en phase solution, le procédé comprenant :
le couplage de l'oxyde de diphénylphosphine benzylique (HOBnDpp) ou de l'oxyde de diphénylphosphine aniline (NH2PhDpp), (i) directement à une molécule de jonction ou (ii) à une molécule de jonction *via* un acide aminé ;
dans lequel l'étape de couplage comprend le couplage de 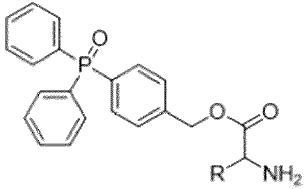
à L-OH pour former un groupe protecteur, le groupe protecteur étant défini par la formule chimique 1 : 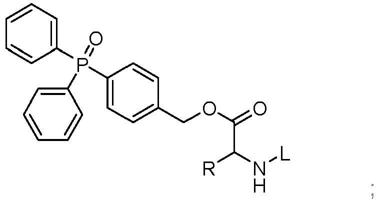 dans laquelle :
R est choisi dans le groupe composé de H, méthyle, -CH₂SH, -CH₂CH₂COOH, - CH₂COOH, benzyle, 4-(1H-imidazolyl)méthyle, -CH(CH₃)(CH₂CH₃), - CH₂CH₂CH₂CH₂NH₂, -CH₂CH₂CH₂NH₂, isobutyle, -CH₂CH₂SCH₃, -CH₂CONH₂, - CH₂CH₂CONH₂, un groupe propyle dans lequel la position 1 du groupe propyle est attachée à la même position que le groupe R et la position 3 du groupe propyle est attachée à l'azote, à -CH₂CH₂CH₂N=C(NH₂)₂, à -CH₂OH, au 1-hydroxyéthyle, à l'isopropyle, au 4-hydroxybenzyle et au 3-(1H-indolyl)méthyle ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

8. Procédé selon la revendication 7, dans lequel l'étape de couplage comprend le couplage de 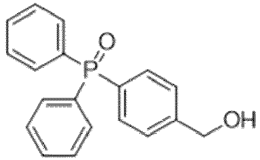 à L-OH pour former un groupe protecteur, le groupe protecteur étant défini par la formule chimique 2 : 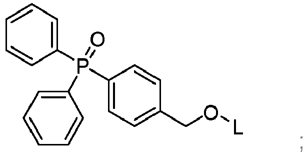 dans laquelle :
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

9. Procédé selon l'une quelconque des revendications 7 ou 8, dans lequel l'étape de couplage comprend le couplage de 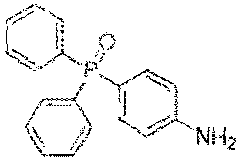 à L-OH pour former un groupe protecteur, le groupe protecteur étant défini par la formule chimique 3 : 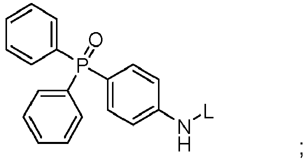 dans laquelle : L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

10. Procédé selon l'une quelconque des revendications 7 à 9, dans lequel l'étape de couplage comprend le couplage de : 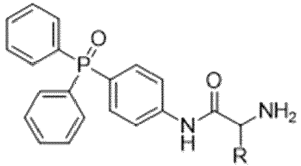 à L-OH pour former un groupe protecteur, le groupe protecteur étant défini par la formule chimique 4 : 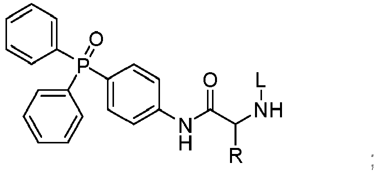 dans laquelle :
R est choisi dans le groupe composé de chaînes latérales connues d'acides aminés protégés et de chaînes latérales connues d'acides aminés non protégés ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

11. Procédé selon l'une quelconque des revendications 7 à 10, dans lequel l'étape de couplage comprend le couplage de 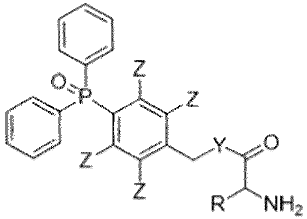 à L-OH pour former un groupe protecteur, le groupe protecteur étant défini par la formule chimique 5 : 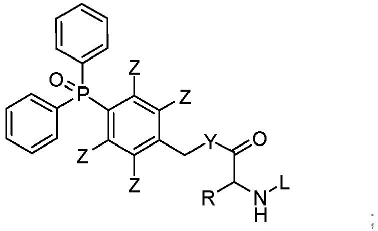 dans laquelle :
Z est choisi dans le groupe composé de : -H, -Me et -OMe ;
Y est choisi dans le groupe composé de : -O-, -S- et -NH- ;
R est choisi dans le groupe composé de chaînes latérales connues d'acides aminés protégés et de chaînes latérales connues d'acides aminés non protégés ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

12. Procédé selon l'une quelconque des revendications 7 à 11, dans lequel l'étape de couplage comprend le couplage de 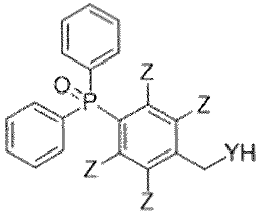 à L-OH pour former un groupe protecteur, où le groupe protecteur est défini par la formule chimique 6 : 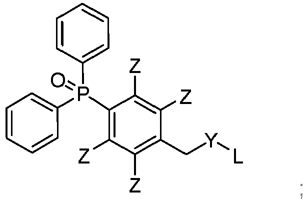
Z est choisi dans le groupe compose de : -H, -Me et -OMe ;
Y est choisi dans le groupe compose de : -O-, -S- et -NH- ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").

13. Procédé selon l'une quelconque des revendications 7 à 12, dans lequel l'étape de couplage comprend le couplage de 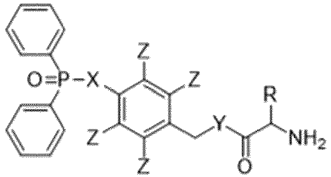 à L-OH pour former un groupe protecteur, le groupe protecteur étant défini par la formule chimique 7 : 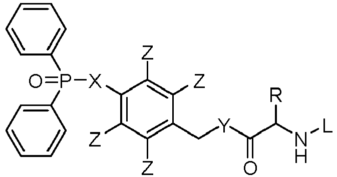 dans laquelle :
Z est choisi dans le groupe composé de : -H, -Me et -OMe ;
Y est choisi dans le groupe composé de : -O-, -S- et -NH- ;
X est choisi dans le groupe composé de : -O-, -S-, and -NH- ;
R est choisi dans le groupe composé de chaînes latérales connues d'acides aminés protégés et de chaînes latérales connues d'acides aminés non protégés ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl") ; et/ou
dans lequel l'étape de couplage comprend le couplage de 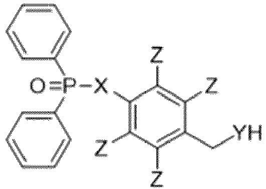
à L-OH pour former un groupe protecteur, le groupe protecteur étant défini par la formule chimique 8 : 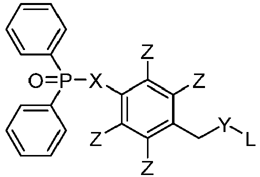 dans laquelle :
Z est choisi dans le groupe composé de : -H, -Me et -OMe ;
Y est choisi dans le groupe composé de : -O-, -S- et -NH- ;
X est choisi dans le groupe composé de : -O-, -S-, et -NH- ; et
L est choisi dans le groupe composé de : 4-(hydroxyméthyl)phénoxyacétyl ("HMPA"), 4-(hydroxyméthyl)phénoxybutanoyl ("HMPB"), 4-(hydroxyméthyl)benzoyl ("HMB"), 4-(mercaptométhyl)benzoyl ("MMB"), 4-(mercaptométhyl)phénoxyacétyl ("MMPA"), 4-(aminométhyl)phénoxyacétyl ("AMPA"), 4-(3,3-diméthyl-3-hydroxypropyl)phénoxyacétyl ("DMPPA"), 2-(4-(amino(2,4-diméthoxyphényl)méthyl)phénoxy)acétyl ("Rink Amide"), 4-((9-amino-9H-xanthen-3-yl)oxy)butanoyl ("Xanthenyl"), 5-(5-amino-10,11-dihydro-5H-dibenzo[a,d][7]annulen-3-yl)pentanoyl ("TCA") et 3-(4-(chloro(2-chlorophényl)(phényl)méthyl)phényl)propanoyl ("2-Chlorotrityl").
